# NEUE EUROPÄISCHE PATENTSCHRIFT

(11) **EP 0 591 914 B2**
(45) Veröffentlichungstag und Bekanntmachung des Hinweises auf die Entscheidung über den Einspruch: **10.02.2010**
(45) Hinweis auf die Patenterteilung: 09.12.1998
(21) Anmeldenummer: 93116058.4
(22) Anmeldetag: 05.10.1993
(51) Int. Cl.: C12N 7/00, C12N 15/48, G01N 33/569, C12Q 1/68, G01N 33/68

(54) **Retrovirus aus der HIV-Gruppe und dessen Verwendung**
Retrovirus of the HIV-group and its application
Rétrovirus du groupe HIV et son application

(30) Priorität: 06.10.1992 DE 4233646; 22.10.1992 DE 4235718; 30.12.1992 DE 4244541; 01.06.1993 DE 4318186
(43) Veröffentlichungstag der Anmeldung: 13.04.1994
(60) Teilanmeldung: 98114623.6
(73) Patentinhaber: Siemens Healthcare Diagnostics Products GmbH, 35041 Marburg (DE)
(72) Erfinder: Gürtler, Lutz G., Prof. Dr., D-81249 München (DE); Eberle, Josef, Dr., D-85356 Freising (DE); Brunn v., Albrecht, Dr., D-86154 Augsburg (DE); Knapp, Stefan, Dr., D-35041 Marburg-Wehrshausen (DE); Hauser, Hans-Peter, Dr., D-35037 Marburg (DE)
(74) Vertreter: Keller, Günter

(56) Entgegenhaltungen:
- EP-A- 0 345 375
- WO-A-87/04459
- WO-A-88/08005
- HUET T. ET AL. NATURE. Bd. 345, 1990, LONDON GB, Seiten 356 - 359, XP000172750
- AGUT H. ET AL. LANCET Bd. 340, September 1992, LONDON UK, Seiten 681 - 682, XP002135358

## Beschreibung

Die vorliegende Erfindung betrifft ein neues Retrovirus aus der HIV-Gruppe sowie Varianten oder Teile davon, welche die wesentlichen Eigenschaften des Virus enthalten. Beschrieben wird ein Verfahren zur Züchtung des Retrovirus. Die Erfindung betrifft weiterhin die Gewinnung dieses Retrovirus sowie die Verwendung des Virus, seiner Teile oder Extrakte für medizinische Zwecke und für die Diagnostik.

Retroviren, die zur sogenannten HIV-Gruppe gehören, führen bei damit infizierten Menschen zu Krankheitserscheinungen, die unter dem Sammelbegriff Immunschwäche bzw. AIDS (Acquired Immune Deficiency Syndrome) zusammengefaßt werden.

Epidemiologische Studien belegen, daá das Humane Immunschwäche Virus (HIV) das aetiologische Agens für die überwiegende Mehrheit der AIDS (Acquired Immune Deficiency Syndrome)-Fälle darstellt. Ein 1983 aus einem Patienten isoliertes und charakterisiertes Retrovirus erhielt die Bezeichnung HIV-1 (Barré-Sinoussi, F. et al., Science 220, 868-871 [1983]). Eine Variante von HIV-1 wird in WO 86/02383 beschrieben.

Eine zweite Gruppe von Humanen Immunschwäche Viren wurde 1985 in Westafrika identifiziert (Clavel, F. et al., Science 233, 343-346 [1986]) und als Humanes Immunschwäche Virus Typ 2 (HIV-2) bezeichnet (EP-A-0 239 425). HIV-2 Retroviren unterscheiden sich deutlich von HIV-1, weisen jedoch auch eine Verwandtschaft zu Affen Immunschwäche Viren (SIV-2) auf. Wie HIV-1 führt auch HIV-2 zu einer AIDS-Symptomatik.

Eine weitere Variante eines Immunschwäche Retrovirus wird in der EP-A-0 345 375 beschrieben und dort als HIV-3 Retrovirus bezeichnet (ANT 70).

Auch in Lancet Vol. 340, Sept. 1992, S. 681-682 wird die Isolierung eines weiteren, varianten Immunschwächevirus beschrieben.

Es ist ein Charakteristikum der Humanen Immunschwäche Viren, daß sie eine hohe Variabilität aufweisen, die die Vergleichbarkeit der verschiedenen Isolate deutlich kompliziert. Beim Vergleich diverser HIV-1-Isolate treten z.B. in einigen Regionen des Genoms hohe Variabilitäten auf, während andere Genombereiche vergleichsweise konserviert vorliegen (Benn, S. et al. Science 230, 949-951 [1985)). Ein wesentlich größerer Polymorphismus konnte auch für HIV-2 beobachtet werden (Clavel, F. et al., Nature 324, 691-695 [1986]). Die größte genetische Stabilität besitzen Bereiche in den gag und pol Genen, die für strukturell und enzymatisch essentielle Proteine codieren; einige Regionen im env-Gen sowie die Gene (vif, vpr, tat, rev, nef), die für regulatorische Proteine codieren, zeigen einen hohen Grad an Variabilität. Es konnte weiterhin gezeigt werden, daß Antisera gegen HIV-1 auch mit gag und pol Genprodukten von HIV-2 kreuzreagieren, obwohl nur geringe Sequenzhomologie vorlag. Ebenfalls war die Hybridisierung zwischen diesen beiden Viren wenig signifikant, wenn nicht sehr wenig stringente Konditionen verwandt wurden (Clavel, F. et al., Nature 324, 691-695 [1986]).

Aufgrund der weiten Verbreitung der Retroviren aus der HIV-Gruppe und der Tatsache, daß zwischen dem Zeitpunkt der Infektion und dem Zeitpunkt, zu dem eindeutige Symptome für pathologische Veränderungen erkennbar sind, ein Zeitraum von einigen bis vielen Jahren (2-20) liegt, ist es epidemiologisch von großer Bedeutung, die Infektion mit Retroviren der HIV-Gruppe möglichst frühzeitig und vor allem zuverlässig zu bestimmen. Dies spielt nicht nur eine Rolle bei der Diagnose von Patienten, die Zeichen von Immunschwäche aufweisen, sondern auch bei der Überprüfung von Blutspendern. Es hat sich herausgestellt, daß bei der Verwendung von Retroviren oder Bestandteilen davon des Types HIV-1 oder HIV-2 in Nachweissystemen bei manchen Seren kein oder nur ein schwacher Nachweis von Antikörpern geführt werden kann, obwohl bei den Patienten, von denen die Seren stammen, Zeichen von Immunschwäche auftreten. Mit Hilfe des erfindungsgemäßen Retrovirus aus der HIV-Gruppe ist in bestimmten Fällen ein derartiger Nachweis möglich.

Beschrieben wird die Isolation und Charakterisierung eines neuen Humanen Immunschwäche Virus, im folgenden als MVP-5180/91 bezeichnet, das aus peripheren Lymphozyten einer 1991 34-jährigen Patientin aus Kamerun isoliert wurde, die Zeichen von Immunschwäche aufwies. Geographisch stammt dieses Retrovirus aus einer Region in Afrika, die zwischen Westafrika mit endemischer HIV-2 und HIV-1 Virusinfektion und Ostzentralafrika mit fast ausschließlicher HIV-1-Verbreitung lokalisiert ist. Offenbart wird also ein neues Retrovirus der HIV-Gruppe, welches als MVP-5180/91 bezeichnet wird und dessen Varianten sowie davon abgeleitete DNS-Sequenzen und Testkits, die davon abgeleitete Aminosäuresequenzen bzw. Teilsequenzen enthalten. Das Retrovirus MVP-5180/91 wurde bei der European Collection of Animal Cell Cultures (ECACC) unter der Nummer V 920 92 318 gemäß den Bedingungen des Budapester Vertrages hinterlegt.

Ebenso wie HIV-1 und HIV-2 wächst das erfindungsgemäße MVP-5180/91 in folgenden Zellinien HUT 78, Jurkat-Zellen, C8166-Zellen und MT-2-Zellen. Die Isolierung und Vermehrung von Viren wird in dem Buch "Viral Quantitation in HIV Infection, Editor Jean-Marie Andrieu, John Libbey Eurotext, 1991" ausführlich beschrieben. Die dort beschriebenen Arbeitsmethoden werden durch Bezugnahme zum Gegenstand der Offenbarung der vorliegenden Anmeldung gemacht.

Weiterhin besitzt das erfindungsgemäße Virus eine magnesiumabhängige Reverse Transkriptase, die aber nicht manganabhängig ist. Dies stellt eine weitere Gemeinsamkeit zu den Viren HIV-1 und HIV-2 dar.

Zum besseren Verständnis der Unterschiede des erfindungsgemäßen MVP-5180/91 Virus zu den Retroviren HIV-1 und HIV-2 soll zunächst kurz der Aufbau der Immunschwäche verursachenden Retroviren erläutert werden. Im Inneren des Virus befindet sich die RNA in einem kegelförmigen Core, das aus Proteinuntereinheiten zusammengesetzt ist, die die Bezeichnung p 24 (p für Protein) tragen. Dieses innere Core wird von einer Proteinhülle umgeben, die aus dem Protein p 17 aufgebaut ist (äußeres Core) und von einer Glykoproteinhülle umgeben ist, die neben Lipiden, die aus der Wirtszelle stammen, das Transmembranprotein gp 41, und das Hüllprotein 120 (gp 120) enthält. Dieses gp 120 kann dann mit den CD-4-Rezeptoren der Wirtszellen eine Bindung eingehen.

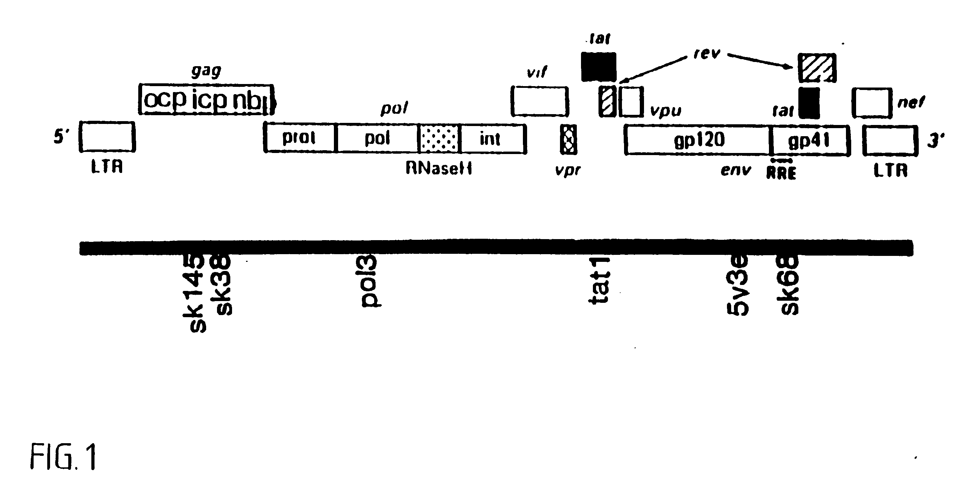

Soweit bekannt, weist die RNA der HIV-Viren - vereinfacht dargestellt - folgende Genbereiche auf: An den beiden Enden sogenannte long terminal repeats (LTR), und die folgenden Genbereiche gag, pol, env und nef. Das Gen gag codiert unter anderem für die Kern(Core)-Proteine, p 24 und p 17, das Gen pol codiert u.a. für die Reverse Transkriptase, die RNAse H und die Integrase und das Gen env codiert für die Glykoproteine gp 41 und gp 120 der Virushülle. Das Gen nef codiert für ein Protein mit Regulatorfunktion. Eine schematische Anordnung des Genomes von Retroviren des HIV-Typs ist in Figur 1 gezeigt.

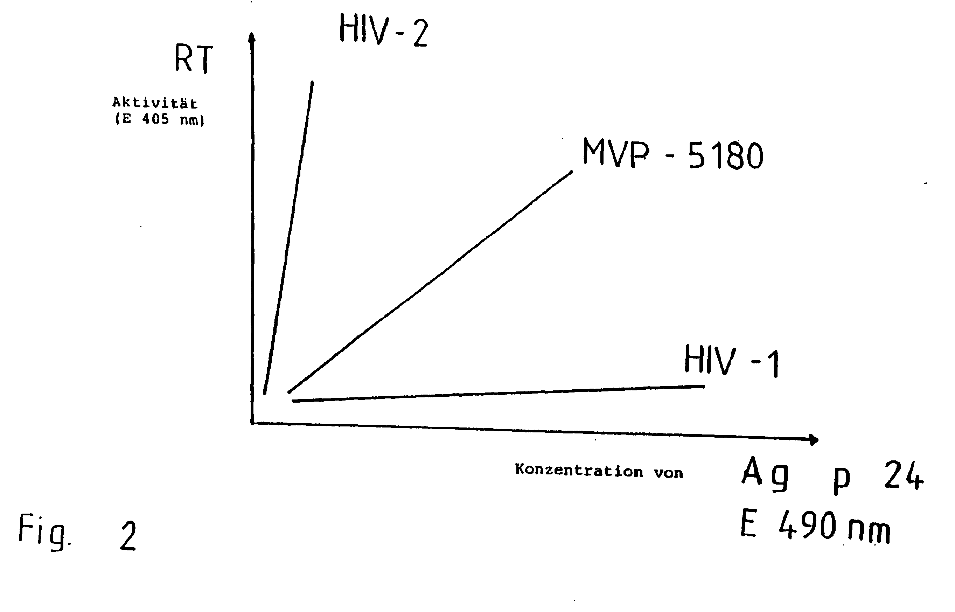

Eine Unterscheidung zwischen den Retroviren HIV-1 und HIV-2 ist u.a. dadurch möglich, daß virales Antigen ausgetestet wird mit einem monoklonalen Antikörper, der kommerziell als Testkit von Abbott (HIVAG-1 Monoclonal) erhältlich ist, und gegen das (HIV-1) p 24 gerichtet ist. Es ist bekannt, daß der Gehalt an Reverser Transkriptase in den Virustypen HIV-1 und HIV-2 etwa gleich ist. Wenn man deshalb in Verdünnungen der aufgeschlossenen Viren die Extinktion (E 490 nm), erhalten durch die Antigen-Antikörper-Reaktion, aufträgt gegen die Aktivität der Reversen Transkriptase, dann erhält man eine Graphik, die etwa der Figur 2 entspricht. Hierbei stellt man fest, daß im Verhältnis zu dem Gehalt an Reverser Transkriptase bei HIV-1 eine sehr hohe Bindungsaffinität für p 24 mit dem eingesetzten monoklonalen Antikörper vorhanden ist. Für HIV-2 tritt dagegen nur eine sehr geringe Bindungsaffinität für p 24 bei Einsatz des monoklonalen Antikörpers wiederum bezogen auf den Gehalt an Reverser Transkriptase auf. Werden diese Messungen durchgeführt für MVP-5180/91, dann befindet sich die Kurve ziemlich genau in der Mitte zwischen der Kurve von HIV-1 und HIV-2, d.h. die Bindungsaffinität des monoklonalen Antikörpers gegen MVP-5180/91 p 24 ist gegenüber HIV-1 reduziert. Figur 2 zeigt schematisch diesen Sachverhalt, wobei RT Reverse Transkriptase bedeutet und als Antigen (Ag) das Protein p 24 eingesetzt wird, gegen das der monoklonale Antikörper, der in dem von Abbott käuflich erwerblichen Testkit vorhanden ist, gerichtet ist.

Ein sehr vielseitig verwendbares System der Gentechnologie ist die sogenannte PCR (polymerase chain reaction) geworden, wobei die zur Durchführung des Verfahrens benötigten Komponenten käuflich erworben werden können. Mit diesem Verfahren ist es möglich, DNA-Sequenzen zu amplifizieren, wenn DNA-Bereiche der zu amplifizierenden Sequenz bekannt sind. Es müssen dann kurze komplementäre DNA-Fragmente (Oligonucleotide = Primer) synthetisiert werden, die sich an einen kurzen Bereich der zu amplifizierenden Nukleinsäuresequenz anlagern. Für die Testdurchführung werden HIV-Nukleinsäuren mit den Primern zusammengebracht in einer Reaktionsmischung, die zusätzlich eine Polymerase und Nukleotidtriphosphate enthält. Die Polymerisation (DNA-Synthese) wird für eine bestimmte Zeit durchgeführt und dann werden die Nukleinsäurestränge durch Erwärmen getrennt. Nach Abkühlen läuft dann die Polymerisation erneut an. Wenn es sich also bei dem erfindungsgemäßen Retrovirus um ein HIV-1 oder HIV-2 Virus handelt, dann müßte die Nukleinsäure amplifiziert werden können, indem Primer verwendet werden, die konserviert sind innerhalb der bekannten Sequenzen der Viren HIV-1 und HIV-2. Derartige Primer sind zum Teil vorbeschrieben (Lauré, F. et al., Lancet ii, (1988) 538-541 für pol 3 und pol 4 bzw. Ou C.Y. et al., Science 239 (1988) 295-297 für sk 38/39, sk 68/69).

Es wurde nun herausgefunden, daß bei Verwendung von bestimmten Primerpaaren, die folgende Sequenz aufweisen:

### (Seq. ID No. 1-14)

### HIV-1

gaga: CTACT AGTAC CCTTC AGG
gagb: CGGTC TACAT AGTCT CTAAA G

sk38: CCACC TATCC CAGTA GGAGA A
sk39: CCTTT GGTCC TTGTC TTATG TCCAG AATGC oder

pol3: TGGGA AGTTC AATTA GGAAT ACCAC
pol4: CCTAC ATAGA AATCA TCCAT GTATT G

pol3n: TGGAT GTGGG TGATG CATA
pol4n: AGCAC ATTGT ACTGA TATCT A sowie

SK145: AGTGG GGGGA CATCA AGCAG CC
SK150: TGCTA TGTCA CTTCC CCTTG GT

145-P: CCATG CAAAT GTTAA AAGAG AC
150-P: GGCCT GGTGC AATAG GCCC
oder eine Kombination von pol3 und pol4 mit
UNI-1: GTGCT TCCAC AGGGA TGGAA
UNI-2: ATCAT CCATG TATTG ATA
(Donehower L.A. et al. (1990) J. Virol. Methods 28, 33-46)
und mit der PCR mit nested primer schwache Amplifikate der MVP-5180/91-DNA erhalten wurden.

Keine oder nur schwache Amplifikate im Vergleich zum HIV-1, die evtl. auf Verunreinigungen zurückzuführen sind, wurden erhalten mit folgenden Primer-Sequenzen:

### (Seq. ID No. 15-34)

tat 1 AATGG AGCCA GTAGA TCCTA
tat 2 TGTCT CCGCT TCTTC CTGCC

tat 1P GAGCC CTGGA AGCAT CCAGG
tat 2P GGAGA TGCCT AAGGC TTTTG

enva: TGTTC CTTGG GTTCT TG
envb: GAGTT TTCCA GAGCA ACCCC
sk68: AGCAG CAGGA AGCAC TATGG
sk69: GCCCC AGACT GTGAG TTGCA ACAG

5v3e: GCACA GTACA ATGTA CACAT GG
3v3e: CAGTA GAAAA ATTCC CCTCC AC
5v3degi: TCAGG ATCCA TGGGC AGTCT AGCAG AAGAA G
3v3degi: ATGCT CGAGA ACTGC AGCAT CGATT CTGGG TCCCC TCCTG AG
3v3longdegi: CGAGA ACTGC AGCAT CGATG CTGCT CCCAA GAACC CAAGG
3v3longext: GGAGC TGCTT GATGC CCCAG A

gagdi: TGATG ACAGC ATGTC AGGGA GT
pol e: GCTGA CATTT ATCAC AGCTG GCTAC

Im Vergleich zum HIV-1 schwache Amplifikate, die jedoch die gleiche Intensität wie das verwendete HIV-2 Isolat (MVP-11971/87) aufwiesen, wurden erhalten mit
gag c: TATCA CCTAG AACTT TAAAT GCATG GG
gag d: AGTCC CTGAC ATGCT GTCAT CA
env c: GTGGA GGGGA ATTTT TCTAC TG
env d: CCTGC TGCTC CCAAG AACCC AAGG.

Eine weitverbreitete Methode zum Nachweis von HIV-Antikörpern ist der sogenannte Western Blot (Immunoblot). Dabei werden die viralen Proteine gelelektrophoretisch aufgetrennt und dann auf eine Membran überführt. Die mit den überführten Proteinen versehenen Membranen werden dann mit Seren der zu untersuchenden Patienten in Verbindung gebracht. Sofern Antikörper gegen die viralen Proteine vorhanden sind, binden diese an die Proteine. Nach Waschen verbleiben lediglich spezifische Antikörper gegen virale Proteine. Die Antikörper werden dann mit Antiantikörpern sichtbar gemacht, die regelmäßig mit einem Enzym gekoppelt sind, das eine Farbreaktion katalysiert. Auf diese Weise können die Banden der viralen Proteine sichtbar gemacht werden.

Das erfindungsgemäße Virus MVP-5180/91 weist gegenüber den Viren HIV-1 und HIV-2 im Western Blot zwei signifikante wesentliche Unterschiede auf. Das HIV-1 zeigt regelmäßig eine starke Bande, die dem Protein p 24 zuzuordnen ist und eine sehr schwache, oft kaum sichtbare Bande, die dem Protein p 23 zuzuordnen ist. HIV-2 weist eine kräftige Bande auf, die dem Protein p 25 zuzuordnen ist und manchmal eine schwache Bande, die dem Protein p 23 zuzuordnen ist. Im Unterschied dazu weist das erfindungsgemäße MVP-5180/91 Virus zwei etwa gleich starke Banden auf, die den Proteinen p 24 und p 25 entsprechen.

Ein weiterer signifikanter Unterschied besteht bei den Banden, die der Reversen Transkriptase zuzuordnen sind. HIV-1 zeigt eine Bande (p 53), die der Reversen Transkriptase entspricht und eine Bande (p 66), die der Reversen Transkriptase verbunden mit der RNAse H entspricht. Bei HIV-2 entspricht die Reverse Transkriptase dem Protein p 55 und, wenn sie mit der RNAse H verbunden ist, dem Protein p 68. Das erfindungsgemäße MPV-5180/91 weist dagegen eine Bande auf bei dem Protein p 48, die der Reversen Transkriptase entspricht, und eine Bande, bei dem Protein p 60, die der Reversen Transkriptase in Verbindung mit RNAse H entspricht.

Aus diesen Ergebnissen kann geschlossen werden, daß die Reverse Transkriptase des MVP-5180/91 ein Molekulargewicht hat, das zwischen etwa 3 und etwa 7 Kilodalton kleiner ist als das der Reversen Transkriptase von HIV-1 bzw. HIV-2. Die Reverse Transkriptase von MPV-5180 weist also ein Molekulargewicht auf, das zwischen etwa 4.500 Dalton und etwa 5.500 Dalton kleiner ist als die Reverse Transkriptase von HIV-1 bzw. HIV-2.

Es wurde herausgefunden, daß mit Hilfe des erfindungsgemäßen Virus MVP-5180/91 anti-env-Antikörper in Seren von deutschen Patienten, die Zeichen von Immunschwäche zeigen, nur schwach nachgewiesen werden können, wobei die Seren aber stark reagieren, wenn anstelle des erfindungsgemäßen Virus ein HIV-1 Virus verwendet wird. Diese stärkere Nachweisreaktion wurde vor allem lokalisiert in dem gp 41 Protein. Bei den Versuchen wurden Serumpanels gegenübergestellt, die einmal von deutschen Patienten stammen und zum anderen von afrikanischen Patienten mit Zeichen von Immunschwäche stammen.

Die oben angegebenen Charakteristika kennzeichnen solche Virus-Varianten, die dem erfindungsgemäßen MVP-5180/91 entsprechen. Wenn also aus heparinisiertem Spenderblut, das von Personen stammt, die Immunschwächeanzeichen aufweisen und vorzugsweise aus Afrika stammen, Immunschwächeviren isoliert werden, dann kann auf diese Weise das erfindungsgemäße Virus oder Varianten davon erhalten werden.

Da das Virus isoliert wurde, welches die oben erwähnten Eigenschaften aufweist, kann die Klonierung einer cDNA auf folgendem Weg durchgeführt werden: Das Virus wird aus einer entsprechend großen Kulturmenge (etwa 1 l) präzipitiert und in phosphatgepufferter Kochsalzlösung aufgenommen. Dann erfolgt eine Pelletierung durch ein (20 %iges) Saccharose-Kissen. Das Viruspellet kann in 6 M Guanidiniumchlorid in 20 mM Dithiothreitol und 0,5 % Nonidet P 40 suspendiert werden. CsCl wird bis auf eine Konzentration von 2 molar zugegeben und die das aufgebrochene Virus enthaltende Lösung wird auf ein Cäsiumchlorid-Kissen aufgebracht. Dann wird die virale RNA durch Zentrifugation pelletiert, gelöst, mit Phenol extrahiert und mit Ethanol und Lithiumchlorid präzipitiert. Mit Hilfe eines Oligo(dT)-Primers wird die Synthese des ersten cDNA-Stranges an der viralen RNA oder Teilen davon durchgeführt. Die Synthese unter Zugabe von Reverser Transkriptase kann durchgeführt werden unter Verwendung eines käuflich erhältlichen Kits. Für die Synthese des zweiten Stranges wird der RNA-Strang des RNA/DNA-Hybrids mit RNase H verdaut und der zweite Strang unter Einsatz von E.coli DNA Polymerase I synthetisiert. Mit Hilfe von T4 DNA Polymerase können dann stumpfe Enden erzeugt werden und diese mit geeigneten Linkern für Restriktionsschnittstellen verbunden werden. Nach Restriktionsverdau mit der geeigneten Restriktionsendonuklease wird das cDNA-Fragment aus einem Agarosegel isoliert und mit einem vorher in geeigneter Weise geschnittenen Vektor ligiert. Der Vektor mit dem cDNA-Insert kann dann zur Transformation von kompetenten E.coli-Zellen verwendet werden. Die erhaltenen Kolonien werden dann auf Membranen übertragen, lysiert und denaturiert und schließlich durch Hybridisierung mit Digoxigenin oder Biotin markierter Nukleinsäure aufgefunden. Nach gentechnologischer Herstellung der entsprechenden cDNA ist eine Isolierung der gewünschten DNA-Fragmente, die aus dem Retrovirus stammen, möglich. Durch Einbau dieser Fragmente in geeignete Expressionsvektoren kann dann das gewünschte Protein bzw. Proteinfragment exprimiert werden und für die diagnostischen Tests eingesetzt werden.

Alternativ zu der angegebenen Methode kann das Immunschwächevirus mit Hilfe der PCR-Technologie kloniert werden, wobei die oben angegebenen Primer Verwendung finden können.

Zwischen verschiedenen Virusisolaten kann die Ähnlichkeit ausgedrückt werden durch den Grad der Homologie der Nucleinsäure- oder Proteinsequenzen. Eine 50 %ige Homologie bedeutet beispielsweise, daß 50 von 100 Nucleotid- oder Aminosäure-Positionen in den Sequenzen übereinstimmen. Die Homologie von Proteinen wird durch Sequenzanalyse bestimmt. Homologe DNA-Sequenzen lassen sich auch durch die Hybridisierungs-Technik ermitteln.

Erfindungsgemäß wurde zunächst ein Teil aus dem Hüllprotein sequenziert und festgestellt, daß diese Sequenz nur eine verhältnismäßig geringe Homologie zu den entsprechenden Sequenzen von Viren vom HIV-Typ aufweist. Insbesondere bezogen auf den gp 41-Bereich wurde durch einen Vergleich mit HIV-Sequenzen, der mit Hilfe von Datenbanken durchgeführt wurde, eine Homologie von höchstens 66 % (Nucleotidsequenz) ermittelt.

Es wurde weiterhin der Bereich sequenziert, der für gp 41 kodiert. Diese Sequenz ist in Tabellen 1 bzw. 3 dargestellt. Der Gegenstand der vorliegenden Erfindung ist in der Anspruchen wiedergegeben.

Gegenstand der vorliegenden Erfindung sind daher solche Viren, die eine Homologie von mehr als 66 %, bevorzugt 75 % und besonders bevorzugt 85 % aufweisen zu dem erfindungsgemäßen HIV-Virus, MVP 5180/91, bezogen auf die Nucleotidsequenz der Tabelle 1 und/oder der Tabelle 3.

Weiterhin sind Gegenstand der vorliegenden Erfindung solche Viren, die eine Homologie von mehr als 66 %, bevorzugt 75 % und besonders bevorzugt 85 % aufweisen zu Partialsequenzen der in Tabelle 3 dargestellten Nucleotidsequenz, die wenigstens 50, bevorzugt 100 Nucleotide lang sind. Dies entspricht einer Länge der Peptide von wenigstens 16 und bevorzugt von wenigstens 33 Aminosäuren.

Das erfindungsgemäße Virus unterscheidet sich durch seine Sequenz von vorbekannten Viren. Offenbart werden daher solche Viren sowie entsprechende DNS- bzw. Aminosäuresequenzen, die der Sequenz des erfindungsgemäßen Virus weitgehend entsprechen, wobei der Grad der Abweichung durch den Grad der Homologie festgelegt wird. Eine Homologie von beispielsweise mehr als 85 % bedeutet daher, daß solche Sequenzen umfaßt werden, die in wenigstens 85 von 100 Nucleotiden bzw. Aminosäuren dieselben Nucleotide bzw. Aminosäuren aufweisen, während der Rest unterschiedlich sein kann. Bei der Feststellung der Homologie werden die beiden Sequenzen derart gegenübergestellt, daß möglichst viele einander entsprechende Nucleotide bzw. Aminosäuren miteinander zur Deckung kommen.

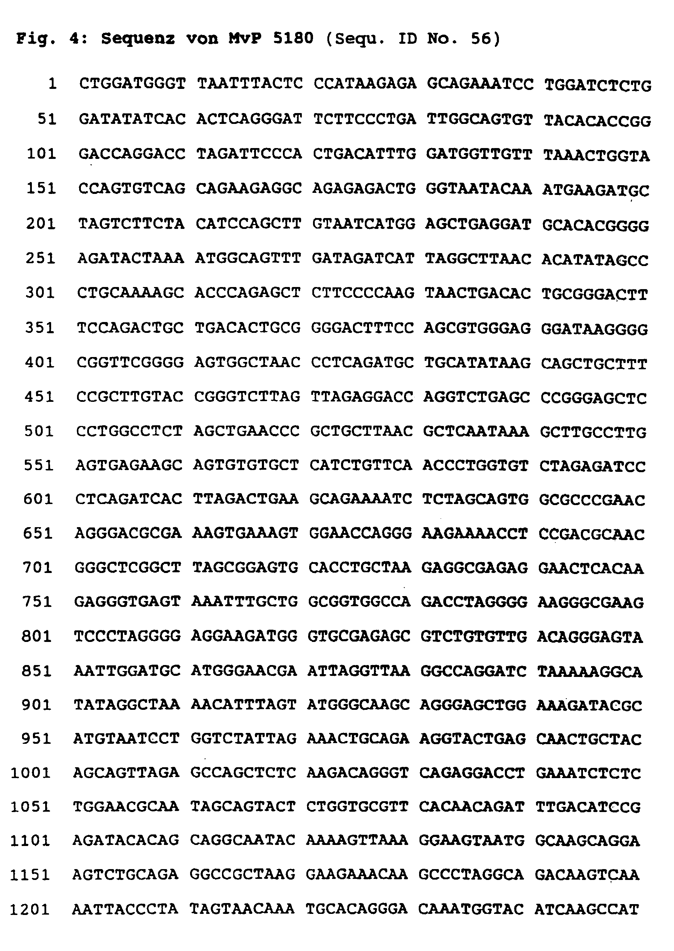

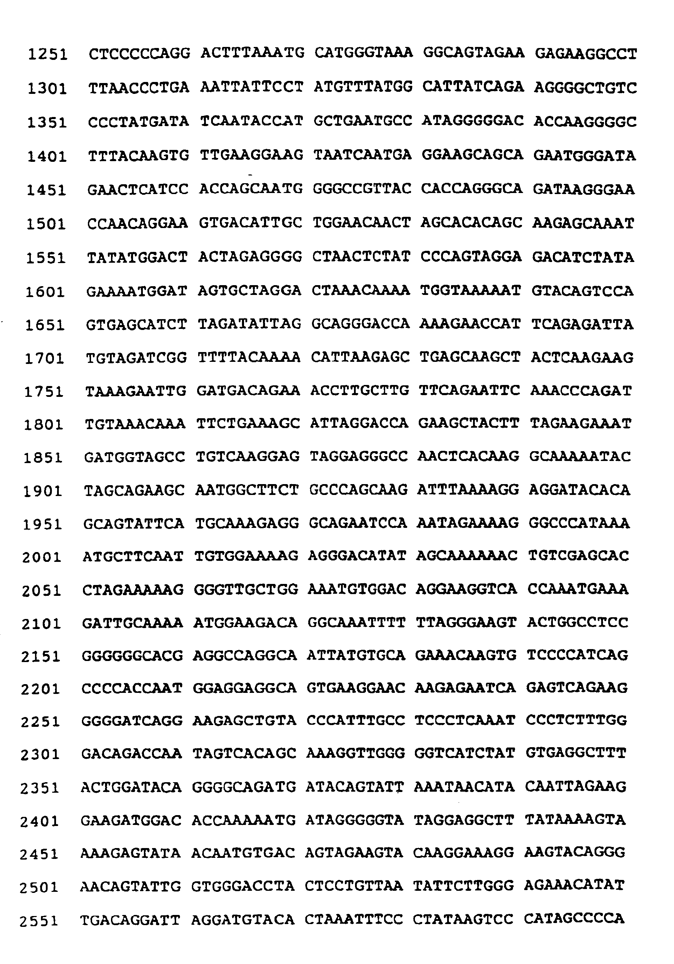

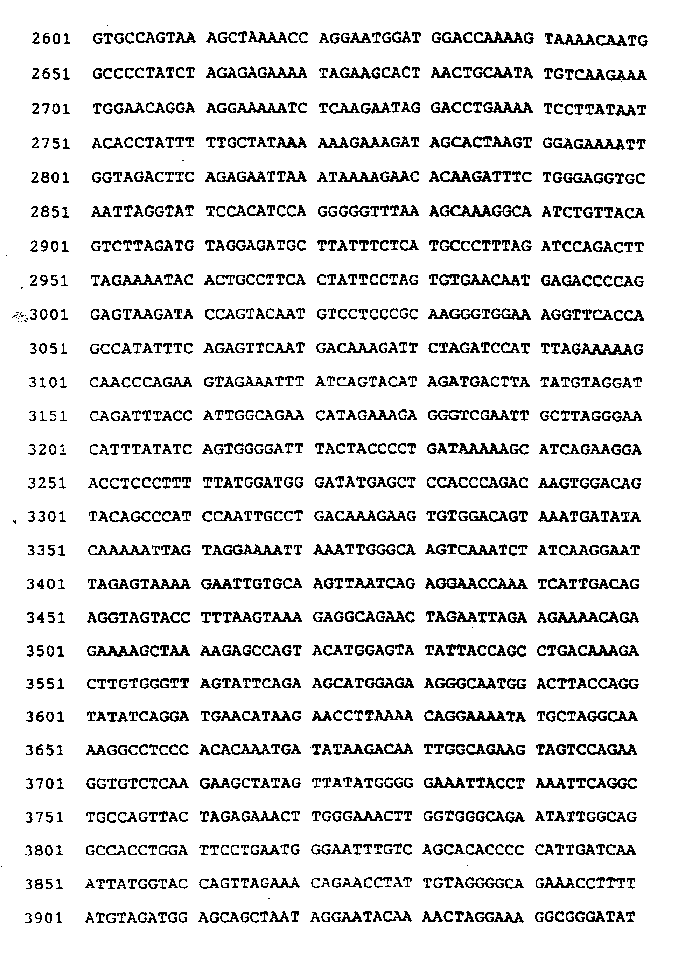

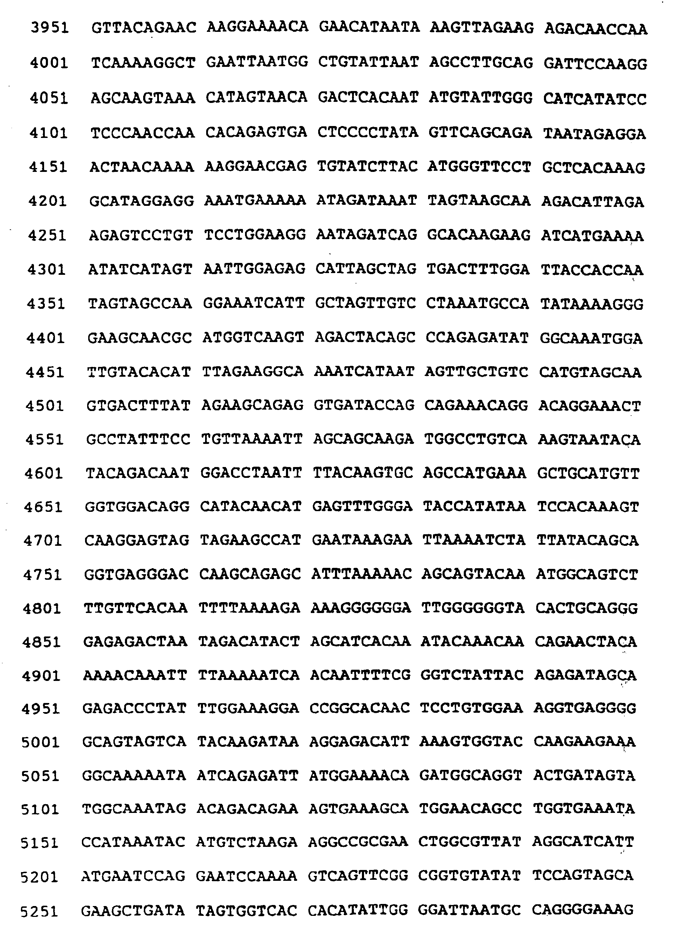

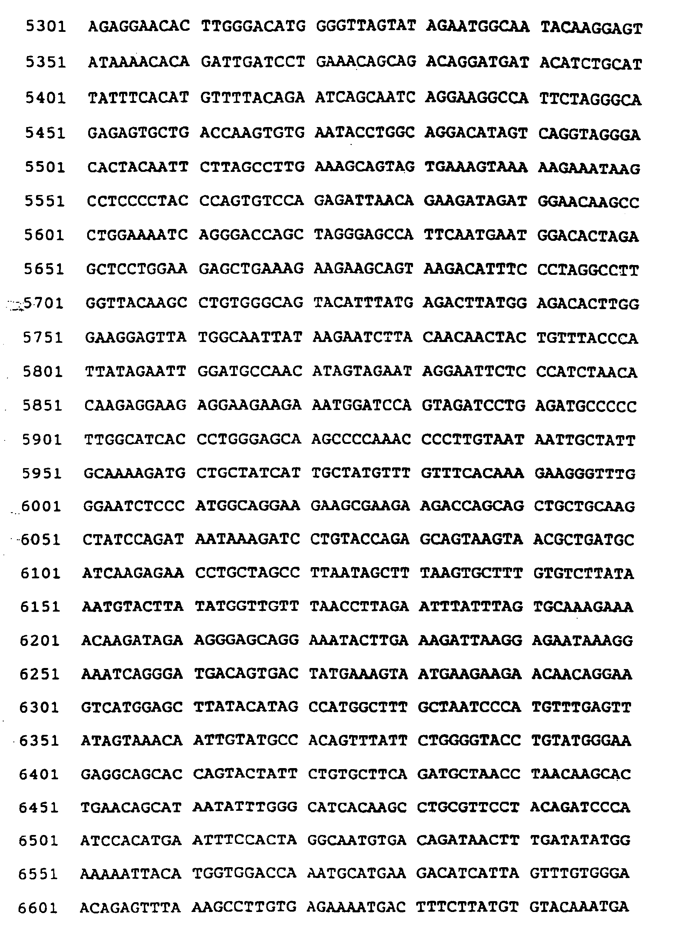

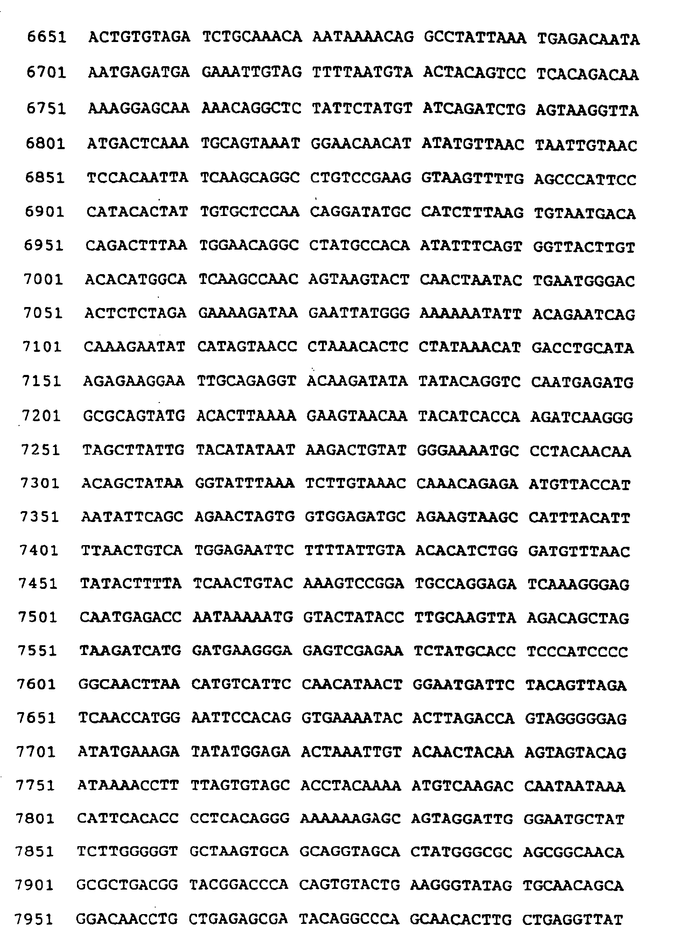

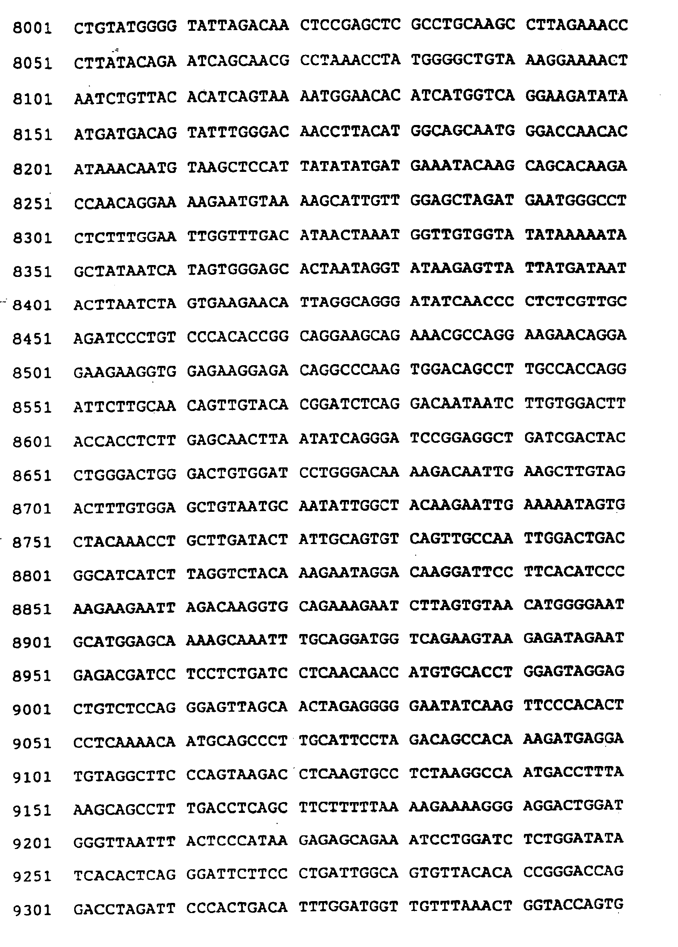

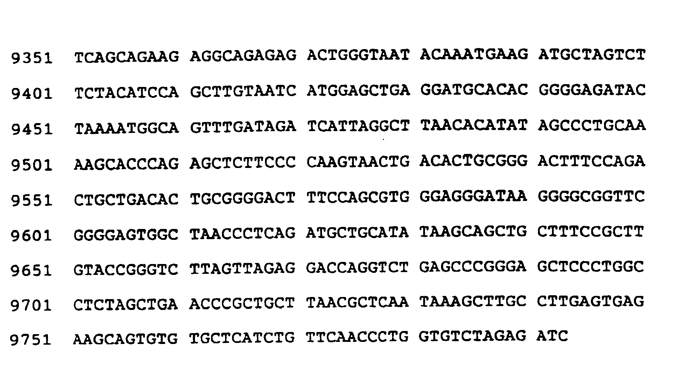

Die (nahezu) vollständige Sequenz, angegeben als DNS-Sequenz des erfindungsgemäßen Virus ist in Fig. 4 wiedergegeben. Offenbart werden dabei Viren, die die Sequenz gemäß Fig. 4 aufweisen sowie Varianten davon, die eine hohe Homologie zu der Sequenz von Fig. 4 aufweisen sowie die diagnostische Verwendung von davon abgeleiteten Proteinen, Polypeptiden und Oligopeptiden.

Anhand der isolierten Sequenz können immundominante Epitope (Peptide) konfektioniert und synthetisiert werden. Da die Nucleinsäuresequenz des Virus bekannt ist, kann der Fachmann hieraus die Aminosäuresequenz ableiten. Ein Teilbereich der Aminosäuresequenz ist in Tabelle 3 angegeben. Offenbart ist daher auch die diagnostische Verwendung von Antigenen, d.h. Proteine, Oligo- oder Polypeptide, die mit Hilfe der in Figur 4 bzw. Tabelle 3 offenbarten Information hergestellt werden können. Diese Antigene, Proteine, Polypeptide und Oligopeptide weisen Aminosäuresequenzen auf, die von Figur 4 abgeleitet werden können bzw. in Tabelle 3 angegeben sind. Die Antigene bzw. Peptide können verhältnismäßig kurze Teilsequenzen einer Aminosäuresequenz aufweisen, die in Tabelle 3 wiedergegeben ist oder aus Figur 4 abgeleitet werden kann. Diese Aminosäuresequenz ist wenigstens 6 Aminosäuren, bevorzugt wenigstens 10 und besonders bevorzugt wenigstens 15 Aminosäuren lang. Hergestellt werden können diese Peptide nicht nur mit Hilfe der rekombinanten Technologie sondern auch durch synthetische Methoden. Ein geeigneter Herstellungsweg ist die Festphasensynthese vom Merrifield-Typ. Eine weitere Beschreibung dieser Technik und anderer im Stand der Technik bekannter Verfahren kann in der Literatur gefunden werden, z.B. M. Bodansky, et al., Peptide Synthesis, John Weeley & Sons, 2nd Edition 1976.

Bei den diagnostischen Tests wird eine Serumprobe der zu untersuchenden Person zusammengebracht mit den Proteinketten von einem oder mehreren Proteinen oder Glykoproteinen (die in eukaryontischen Zellinien exprimiert werden können) oder Teilen davon, die von MVP-5180/91 stammen. Bevorzugte Testverfahren schließen die Immunfluoreszenz oder immunenzymatische Testverfahren (z.B. Elisa, Immunoblot) ein.

Bei den immunenzymatischen Tests (ELISA) kann beispielsweise Antigen, das von MVP-5180/91 oder einer Variante davon stammt, an den Wänden von Mikrotiterplatten gebunden werden. Die dabei verwendete Dosierung hängt von dem Testsystem und der Behandlung der Mikrotiterplatten wesentlich ab. Dann wird Serum bzw. Serumverdünnungen, die von der zu untersuchenden Person stammen, in die Löcher der Mikrotiterplatten gegeben. Nach einer bestimmten Inkubationszeit wird die Platte gewaschen und spezifische Immunkomplexe werden nachgewiesen durch Antikörper, die spezifisch an menschliche Immunglobuline binden und die vorher mit einem Enzym, beispielsweise Meerrettichperoxidase, alkalischer Phosphatase usw. verbunden wurden oder mit enzymmarkiertem Antigen. Diese Enzyme können ein farbloses Substrat in ein stark gefärbtes Produkt umwandeln und an der Stärke der Färbung kann dann das Vorhandensein von spezifischen Anti-HIV-Antikörpern abgelesen werden. Eine weitere Möglichkeit der Verwendung des erfindungsgemäßen Virus in Testsystemen ist die Verwendung in Western Blots.
Auch wenn die Herstellung von Impfstoffen gegen Immunschwächeerkrankungen sich als äußerst schwierig erweist, kann doch auch dieses Virus bzw. Teile davon, d.h. immundominante Epitope und Induktoren der zellulären Immunität, oder gentechnologisch hergestellte Antigene zur Entwicklung und Herstellung von Impfstoffen verwendet werden.

### Beispiel 1

Das erfindungsgemäße Immunschwäche-Virus MVP-5180/91 wurde aus dem Blut einer Patientin mit Zeichen von Immunschwäche isoliert. Hierzu wurden periphere mononukleäre Zellen (peripheral blood lymphocytes, PBL) und periphere Lymphozyten aus dem Blut (PBL) eines nicht HIV-infizierten Spenders mit Phytohämagglutinin stimuliert und in Kultur gehalten. Verwendet wurde hierzu das übliche Medium RPMI 1640 mit 10 % fötalem Kälberserum. Die Kulturbedingungen sind beschrieben in Landay A. et al., J. Inf. Dis., 161 (1990) S. 706-710. Beobachtet wurde dann die Bildung von Riesenzellen unter dem Mikroskop. Die Produktion von HIV-Viren wurde über die Bestimmung des p 24-Antigens mit Hilfe des käuflich erwerbbaren Tests von Abbott bestimmt. Ein weiterer Test zur Bestimmung des Wachstums der Viren war der Test unter Verwendung von partikelgebundener Reverser Transkriptase (Eberle J., Seibl R., J. Virol. Methods 40, 1992, S. 347-356). Das Wachstum der Viren wurde also anhand der enzymatischen Aktivitäten im Kulturüberstand ein- bis zweimal pro Woche bestimmt, um die Virusproduktion zu überwachen. Wöchentlich einmal wurden neue Spenderlymphozyten zugegeben.

Nachdem eine HIV-Virenvermehrung festgestellt werden konnte, wurden frische periphere Lymphozyten aus dem Blut (PBL) nicht HIV-infizierter, gesunder Spender mit dem Überstand der ersten Kultur infiziert. Dieser Schritt wurde wiederholt und dann wurden mit dem Überstand H 9 bzw. HUT 78 Zellen infiziert. Auf diese Weise war eine permanente Produktion des Immunschwäche-Virus möglich. Das Virus wurde bei der ECACC unter der Nr. V 920 92 318 hinterlegt.

### Beispiel 2

Zum Nachweis von HIV-Infektionen ist derzeit der sogenannte Western Blot oder Immunoblot ein Standardverfahren. Gemäß der in J. Virol. Meth. 15 (1987) S. 11-23 von Gürtler et al. beschriebenen Vorgehensweise wurden verschiedene Seren untersucht. Hierbei wurden Seren von deutschen Patienten solchen Seren gegenübergestellt, die von afrikanischen Patienten erhalten wurden. Es wurden hierbei folgende Ergebnisse erhalten:

| Virustyp | deutsche Seren | afrikanische Seren |
|---|---|---|
| HIV-1, Virus isoliert von deutschem Patienten | starke Reaktion | starke Reaktion mit gp 41 |
| MVP-5180/91 | keine bis schwache Reaktion mit gp 41 | starke Reaktion |

Die oben dargestellten Ergebnisse zeigen, daß ein aus deutschen Patienten isoliertes Virus vom HIV-1 Typ, wenn es zum Nachweis von HIV-Infektionen verwendt wird, möglicherweise keine eindeutigen Ergebnisse liefert, wenn der Patient infiziert wurde mit einem Virus, das dem erfindungsgemäßen MVP-5180/91 entspricht. Es wird dabei davon ausgegangen, daß mit Hilfe des erfindungsgemäßen Virus solche Viren nachgewiesen werden können, die wenigstens etwa 85 % Homologie, bezogen auf das Gesamtgenom, zu dem erfindungsgemäßen Virus aufweisen.

### Beispiel 3

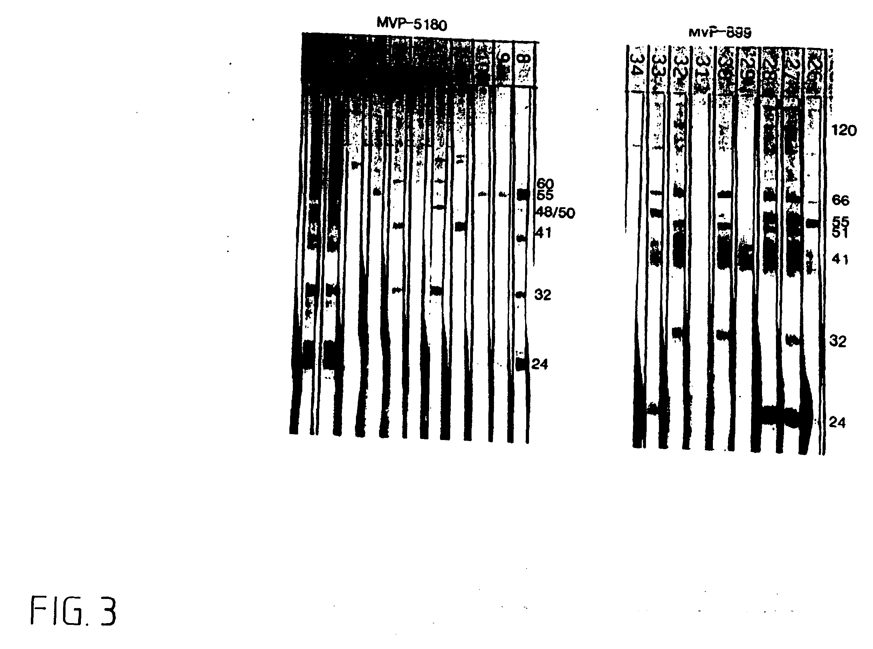

Gemäß der in Beispiel 2 angegebenen Vorgehensweise wurden weitere Western Blots durchgeführt. Die Ergebnisse sind in der anliegenden Figur 3 dargestellt. Bei diesem Test wurde einmal das virale Protein des erfindungsgemäßen Immunschwäche-Virus MVP-5180/91 und einmal das virale Protein eines HIV-1-Typ Virus (MVP-899) gelelektrophoretisch aufgetrennt und dann auf Zellulosefilter transferiert. Diese Filterstreifen wurden mit den Seren von verschiedenen Patienten inkubiert und dann wurden die spezifischen Antikörper durch eine Farbreaktion sichtbar gemacht. Die linke Hälfte der Figur mit der Überschrift MVP-5180 zeigt das erfindungsgemäße Immunschwäche-Virus. Die rechte Hälfte der Figur zeigt ein aus deutschem Spender isoliertes Virus (MVP-899), bei dem es sich um ein HIV-1-Virus handelt.

Die einzelnen Filterstreifen wurden nun mit den Seren von verschiedenen Patienten inkubiert. Betrachtet man die Figur 3, so wurden jeweils dieselben Seren (von deutschen Patienten) umgesetzt mit je zwei Filterstreifen, wobei die Nummern 8 und 26; 9 und 27; 10 und 28; 11 und 29; 12 und 30; 13 und 31; 14 und 32; 15 und 33 sowie 16 und 34 die gleichen Seren bezeichnen. Bei den Western Blots mit den Nummern 17 und 18 wurden Seren von afrikanischen Patienten eingesetzt. Die Zahlen an den rechten Seitenrändern geben die annähernden Molekulargewichte in Tausend (KD) an.

Die Figur 3 zeigt deutlich, daß Seren von deutschen Patienten mit dem erfindungsgemäßen Immunschwäche-Virus im Western Blot mit dem gp 41 nur sehr schwach reagieren. Seren von afrikanischen Patienten dagegen reagieren mit dem erfindungsgemäßen Immunschwäche-Virus sehr stark. Figur 3 macht daher deutlich, daß unter Verwendung des erfindungsgemäßen Immunschwäche-Virus solche Immunschwäche-Infektionen nachgewiesen werden können, die bei Verwendung eines HIV-1 oder HIV-2-Virus nur fragliche, also nicht eindeutig positive Ergebnisse liefern. Diese Nachweismöglichkeit kann weitreichende diagnostische Bedeutung haben, da in den Fällen, in denen nur fragliche Ergebnisse im Western Blot erzielt werden, nicht mit eindeutiger Sicherheit festgestellt werden kann, ob es sich um eine Infektion mit einem Immunschwäche-Virus handelt. Wenn aber mit Hilfe des erfindungsgemäßen Immunschwäche-Virus derartige fragliche Ergebnisse einer Infektion mit einem Virus des erfindungsgemäßen Typs zugeordnet werden können, dann stellt dies einen erheblichen diagnostischen Fortschritt dar.

### Beispiel 4

### DNA-Isolierung, Amplifizierung und strukturelle Charakterisierung von Genomabschnitten des HIV-Isolates MVP-5180/91

Genomische DNA aus MVP-5180/91-infizierten HUT 78-Zellen wurden nach Standardmethoden isoliert.

Zur Charakterisierung von Genombereichen des Isolates MVP-5180/91 wurden PCR (Polymerase Chain Reaction)Experimente mit einem Primerpaar aus dem Hüllproteinbereich gp 41 durchgeführt. Die Durchführung der PCR-Experimente erfolgte nach der Methode von Saiki et al. (Saiki et al., Science 239: 487-491, 1988) mit folgenden Modifikationen: Für die Amplifikation HIV-spezifischer DNA-Bereiche wurden 5 µl genomische DNA aus MVP-5180/91 infizierten HUT 78-Zellen in einem 100 µl Reaktionsansatz (0,25 mM dNTP, je 1 µM Primer 1 und Primer 2, 10 mM Tris HCl pH 8,3, 50 mM KCl, 1,5 mM MgCl₂, 0,001 % Gelatine, 2,5 units Taq Polymerase (Perkin Elmer) pipettiert und nach folgendem Temperatur-Programm amplifiziert: 1. Initiale Denaturierung: 3' 95°C, 2. Amplifikation: 90'' 94°C, 60'' 56°C, 90'' 72°C (30 Cyclen).

Die für die PCR und die Nucleotidsequenzierung verwendeten Primer wurden auf dem Oligonucletidsynthesizer 8750 der Firma Biosearch synthetisiert (Seq. ID No. 35 + 36).
Primer 1: AGC AGC AGG AAG CAC TAT GG (Koordinaten aus HIV 1 Isolat HXB2: Base 7795-7814, entspricht Primer sk 68)
Primer 2: GAG TTT TCC AGA GCA ACC CC (Koordinaten aus HIV 1 Isolat HXB2: Base 8003-8022, entspricht Primer env b).

Die amplifizierte DNA wurde über ein 3 % "Nusieve"-Agarosegel (Fa. Biozyme) aufgetrennt, das amplifizierte Fragment ausgeschnitten und mit dem gleichen Volumen an Puffer (1xTBE (0,09 M TrisBorat, 0,002 M EDTA pH8,0) versetzt. Nach Inkubation des DNA-Agarosegemisches für 10 Minuten bei 70°C und nachfolgender Phenolextraktion wurde die DNA aus der wässrigen Phase durch Zugabe von 1/10 Vol 3M NaAc pH 5,5 und 2 Vol Ethanol bei -20°C 15' gefällt und anschließend in einer Zentrifuge (Eppendorf) pelletiert (13000 rpm, 10', 4°C). Die pelletierte DNA wurde getrocknet, in Wasser aufgenommen und nach der photometrischen Bestimmung der DNA-Konzentration bei 260 nm im Spektralphotometer (Beckman) nach der Methode von Sanger (F. Sanger, Proc. Natl.Adac.Sci., 74: 5463, 1977) sequenziert. Anstelle der Sequenzierung mit Klenow DNA Polymerase, wurde die Sequenzierungsreaktion mit einem Kit von Applied Biosystems ("Taq Dye Deoxy Terminator Cycle Sequencing", Best.-Nr.: 401150) durchgeführt. Als Primer wurden in getrennten Sequenzierungsreaktionen Primer 1 oder Primer 2 (jeweils 1 µM) eingesetzt. Die Analyse der Sequenzierungsreaktion erfolgte auf dem DNA-Sequenziergerät 373A (Applied Biosystems) nach den Vorgaben des Geräteherstellers.

Die Nucleotidsequenz des amplifizierten DNA-Bereichs und die davon abgeleitete Aminosäuresequenz ist in der Tabelle 1 dargestellt (Sequ. ID No. 37-39).

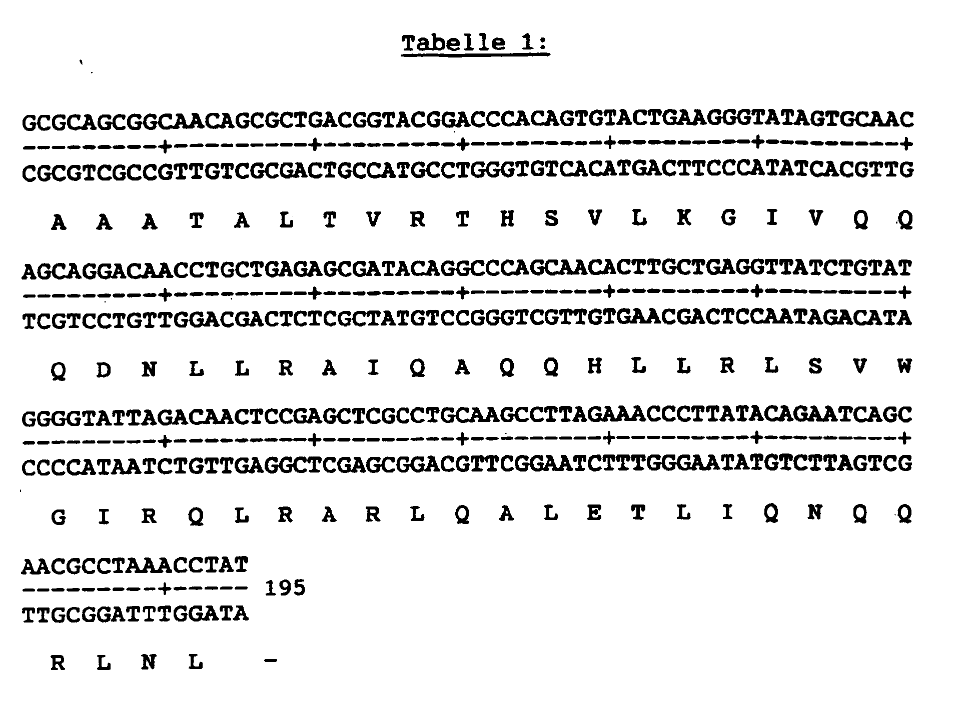

### Beispiel 5

Die gefundene Nucleotidsequenz aus Tabelle 1 wurde auf homologe Sequenzen in der GENEBANK-Datenbank (Release 72, Juni 1992) mit Hilfe des GCG-Computerprogramms (Genetic Computer Group, Inc., Wisconsin USA, Version 7.1, März 1992) untersucht. In dieser Datenbank sind die meisten der bis Juli 1992 bekannten Nucleotidsequenzen von Immundefizienzviren humanen Ursprungs und von Isolaten aus Primaten enthalten.

Die Nucleotidsequenz aus Tabelle 1 weist im besten Fall eine 66 %ige Homologie zu einem Schimpansen-Isolat auf. Zu HIV 1-Isolaten ist MVP 5180/91 in der untersuchten DNA-Sequenz im besten Fall zu 64 % homolog. Zu HIV 2-Isolaten ist die DNA aus Tabelle 1 zu 56 % homolog. Außer der Sequenz des Schimpansenisolates besteht die beste Homologie zwischen der Nucleotidsequenz aus Tabelle 1 und DNA-Abschnitten aus Primatenisolaten (SIV: Simian Immunodeficiency Virus) in einer DNA-Sequenz, die für einen Teilbereich des Hüllproteins des Isolates SIV (Afrikanische Meerkatze) TYO-1 kodiert. Die Homologie beträgt 61,5 %.

### Beispiel 6

Die gefundene Aminosäuresequenz aus Tabelle 1 wurde auf homologe Sequenzen in der SWISSPROT Protein-Datenbank (Release 22, Juni 1992) mit Hilfe des GCG-Computerprogramms untersucht. In dieser Datenbank sind die meisten der bis Juni 1992 bekannten Proteinsequenzen von Immundefizienz-Viren humanen Ursprungs und von Isolaten aus Primaten enthalten.

Die Aminosäuresequenz aus Tabelle 1 ist im besten Fall mit 62,5 % zu einem Hüllproteinabschnitt des oben genannten Schimpansenisolates homolog. Unter HIV 1-Hüllproteinen findet man die beste Homologie mit der Aminosäuresequenz aus Tabelle 1, in dem Isolat HIV 1 Mal. Die Homologie beträgt 59 %. Zu HIV 2-Hüllproteinen beträgt die Homologie mit der Aminosäuresequenz aus Tabelle 1 im besten Fall 52 % (Isolat HIV 2 Rod). Da auch HIV 1 und HIV 2-Isolate im besten Fall im korrespondierenden Proteinabschnitt nur zu 64 % identisch sind, scheint es sich bei dem Isolat MVP-5180/91 um eine HIV-Variante zu handeln, die sich deutlich von HIV 1 und von HIV 2 strukturell abgrenzt und somit einen Vertreter einer davon unabhängigen Gruppe von HIV-Viren repräsentiert.

Die Aminosäuresequenz des amplifizierten DNA-Bereiches (Tabelle 1) des HIV-Isolates MVP 5180/91 überlappt mit einem immundiagnostisch wichtigen Bereich aus dem Hüllprotein gp 41 von HIV 1 (Aminosäure 584-618∗) (Tabelle 2) (Gnann et al., J. Inf. Dis. 156: 261-267, 1987; Norrby et al., Nature, 329: 248-250, 1987).

Korrespondierende Aminosäurebereiche aus den Hüllproteinen von HIV 2 und SIV sind ebenfalls immundiagnostisch konserviert (Gnann et al., Science, S. 1346-1349, 1987). So werden Peptide aus diesem Hüllproteinbereich von HIV 1 und HIV 2 in vielen kommerziell erhältlichen HIV 1/2 Antikörper-Screeningtests als Festphasenantigene eingesetzt. Ungefähr 99 % der anti HIV 1 und anti HIV 2 positiven Seren können damit erfaßt werden.

Der Aminosäurebereich des MVP-5180/91-Hüllproteins (Tabelle 1) könnte aufgrund der Überlappung mit dem immundiagnostisch wichtigen Bereich aus gp 41 serodiagnostisch von Bedeutung sein. Dies wäre insbesondere dann der Fall, wenn Antiseren von HIV-infizierten Patienten mit keinem der kommerziell erhältlichen Antikörper-Screeningtests positiv reagieren würden. In diesen Fällen könnte eine Infektion mit einem MVP-5180/91 eng verwandten Virus vorliegen.

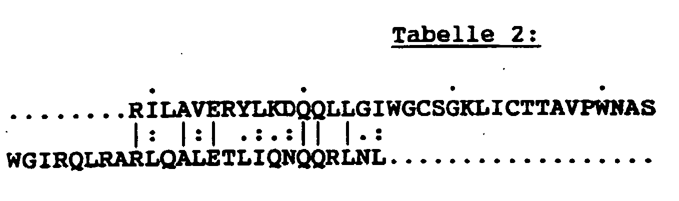

### Beispiel 7

### DNA Isolierung, Amplifizierung und strukturelle Charakterisierung von Genomabschnitten des HIV Isolates MVP-5180/91 (kodierend für gp 41)

Genomische DNA aus MVP-5180/91-infizierten HUT78-Zellen wurden wie beschrieben isoliert.

Zur Charakterisierung von Genombereichen des Isolates MVP-5180/91 wurden PCR (Polymerase Chain Reaction)-Experimente mit Primerpaaren aus dem Hüllproteinbereich gp 41 durchgeführt. Die PCR (Saiki et al., Science 239: 487-491, 1988) und inverse PCR (Triglia et al., Nucl. Asids, Res. 16: 8186, 1988) wurden mit folgenden Modifikationen durchgeführt:

### 1. PCR

Für die Amplifikation HIV-spezifischer DNA Bereiche wurden 5 µl (218 µg/ml) genomische DNA aus MVP-5180/91 infizierten HUT78 Zellen in einem 100 µl Reaktionsansatz (0,25 mM dNTP, je 1 µM Primer 163env und Primer envend, 10 mM Tris HCl pH 8.3, 50 mM KCl, 1.5 mM MgCl₂, 0,001 % Gelatine, 2,5 units Taq Polymerase (Perkin Elmer)) pipettiert und nach folgendem Temperaturprogramm amplifiziert: 1. Initiale Denaturierung: 3 min. 95°C, 2. Amplifikation: 90 sec. 94°C, 60 sec. 56°C, 90 sec. 72°C (30 Cyclen).

### 2. Inverse PCR

Der 5' Bereich von gp 41 (N-Terminus) und die 3' Sequenz von gp 120 wurden mittels "inverser PCR" amplifiziert. Hierzu wurden 100 µl einer genomischen DNA Präparation (218 µg/ml) aus MVP-5180/91-infizierten HUT78-Zellen in einem Endvolumen von 200 µl mit 10 units der Restriktionsendonuklease Sau3a 1 Stunde bei 37°C verdaut. Die DNA wurde anschließend phenolisiert und mit Natriumazetat (Endkonzentration 300 mM) und 2.5 Volumen Ethanol 10 min bei -70°C gefällt, in der Eppendorfzentrifuge abzentrifugiert und das Pellet getrocknet und in 890 µl Aqua dest. resuspendiert. Nach Zugabe von 100 µl Ligasepuffer (50 mM Tris HCl, pH 7,8, 10 mM MgCl₂, 10 mM DTT, 1 mM ATP, 25 µg/ml Rinderserumalbumin) und 10 µl T4 DNA-Ligase (Fa. Boehringer, Mannheim) wurden die DNA-Fragmente 3 Stunden bei Raumtemperatur ligiert, erneut phenolisiert und mit Natriumazetat und Ethanol wie oben gefällt. Nach dem Abzentrifugieren und Trocknen wurde die DNA in 40 µl Aqua dest. resuspendiert und mit 10 units der Restriktionsendonuklease SacI (Fa. Boehringer, Mannheim) für 1 Stunde verdaut. Anschließend wurden 5 µl dieses Ansatzes in einem PCR-Experiment wie unter "1. PCR" dargestellt, eingesetzt. Anstelle der Primer 163env und envend wurden für die inverse PCR die Primer 168i und 169i verwendet.

Die Primer 163env, 168i und 169i wurden aus der bereits ermittelten Teilsequenz des HIV-Isolates MVP-5180 ausgewählt (Beispiel 4).

Die für die PCR/inverse PCR und die Nucleotidsequenzierung verwendeten Primer wurden auf dem Oligonucleotidsynthesizer 8750 der Firma Biosearch synthetisiert, wobei die Primer folgende Sequenzen aufwiesen (Sequ. ID No. 40-43):
Primer 163env: 5' CAG AAT CAG CAA CGC CTA AAC C 3'
Primer envend: 5' GCC CTG TCT TAT TCT TCT AGG 3' (Position aus HIV 1 Isolat BH10: Base 8129-8109)
Primer 168i: 5' GCC TGC AAG CCT TAG AAA CC 3'
Primer 169i: 5' GCA CTA TAC CCT TCA GTA CAC TG 3'

Die amplifizierte DNA wurde über ein 3 % "Nusieve"-Agarosegel (Fa. Biozyme) aufgetrennt, das amplifizierte Fragment ausgeschnitten und mit dem gleichen Volumen an Puffer (1xTBE (0.09 M TrisBorat, 0.002 M EDTA, pH 8.0)) versetzt. Nach Inkubation des DNA-Agarosegemisches für 10 Minuten bei 70°C und nachfolgender Phenolextraktion wurde die DNA aus der wässrigen Phase durch Zugabe von 1/10 Vol 3M NaAc, pH 5,5 und 2 Vol Ethanol bei -20°C 15' gefällt und anschließend in einer Eppendorfzentrifuge pelletiert (13000rpm, 10', 4°C). Die pelletierte DNA wurde getrocknet, in Wasser aufgenommen und nach der photometrischen Bestimmung der DNA-Konzentration bei 260nm im Spektralphotometer (Fa. Beckman) nach der Methode von Sanger (F. Sanger, Proc. Natl. Acad. Sci., 74:5463, 1977) sequenziert. Anstelle der Sequenzierung mit Klenow DNA Polymerase wurde die Sequenzierungsreaktion mit einem Kit der Fa. Applied Biosystems ("Taq Dye Deoxy Terminator Cycle Sequencing", Best. Nr.: 401150) durchgeführt. Als Primer wurden in getrennten Sequenzierungsreaktionen Primer 163env oder Primer envend (jeweils 1µM) eingesetzt. Die amplifizierte DNA aus dem inversen PCR-Experiment wurde mit den Primern 168i und 169i sequenziert. Die Analyse der Sequenzierungsreaktion erfolgte auf dem DNA-Sequenziergerät 373A (Applied Biosystems) nach den Vorgaben des Geräteherstellers.

Die Nucleotidsequenz des amplifizierten DNA-Bereichs und die davon abgeleitete Aminosäuresequenz sind in der Tabelle 3 dargestellt (Sequ. ID No. 44-46).

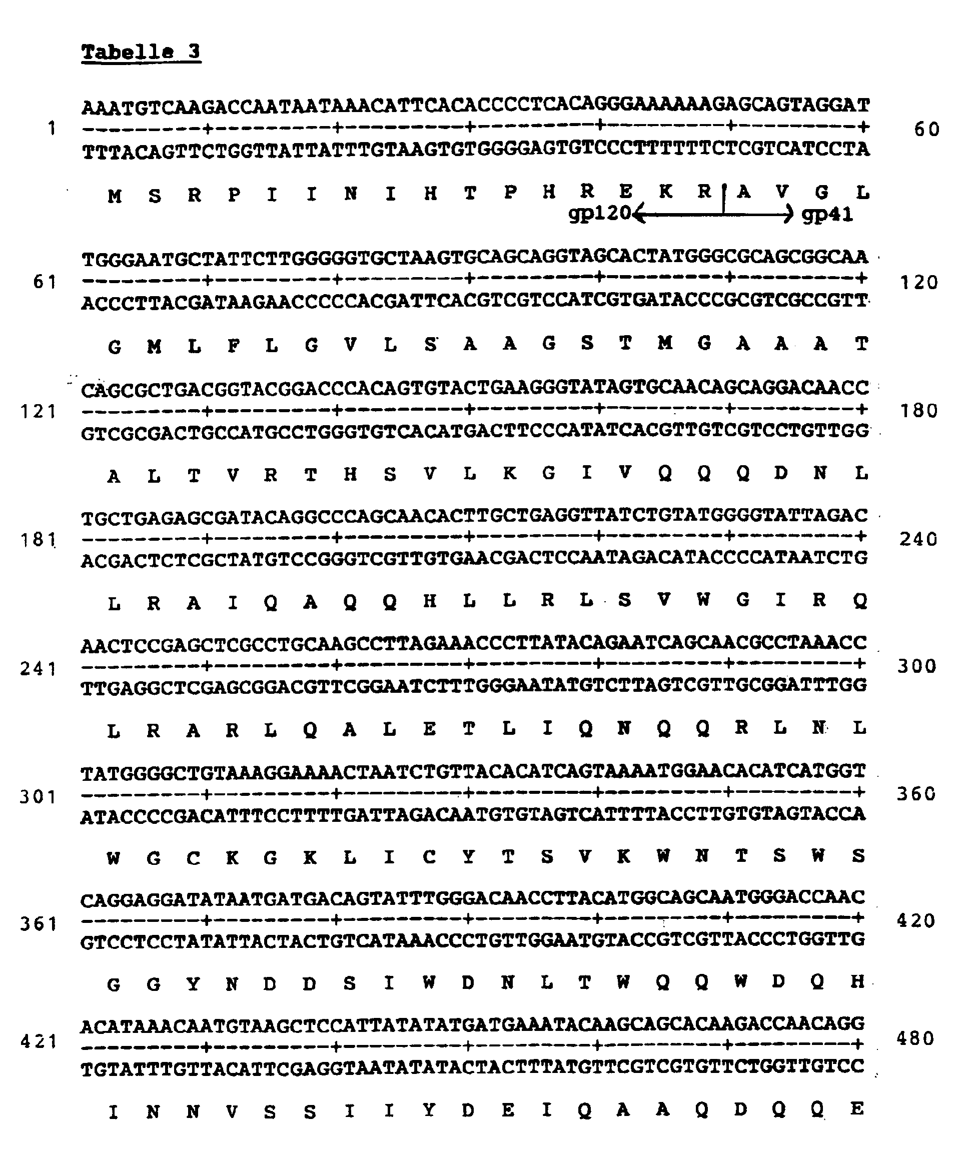

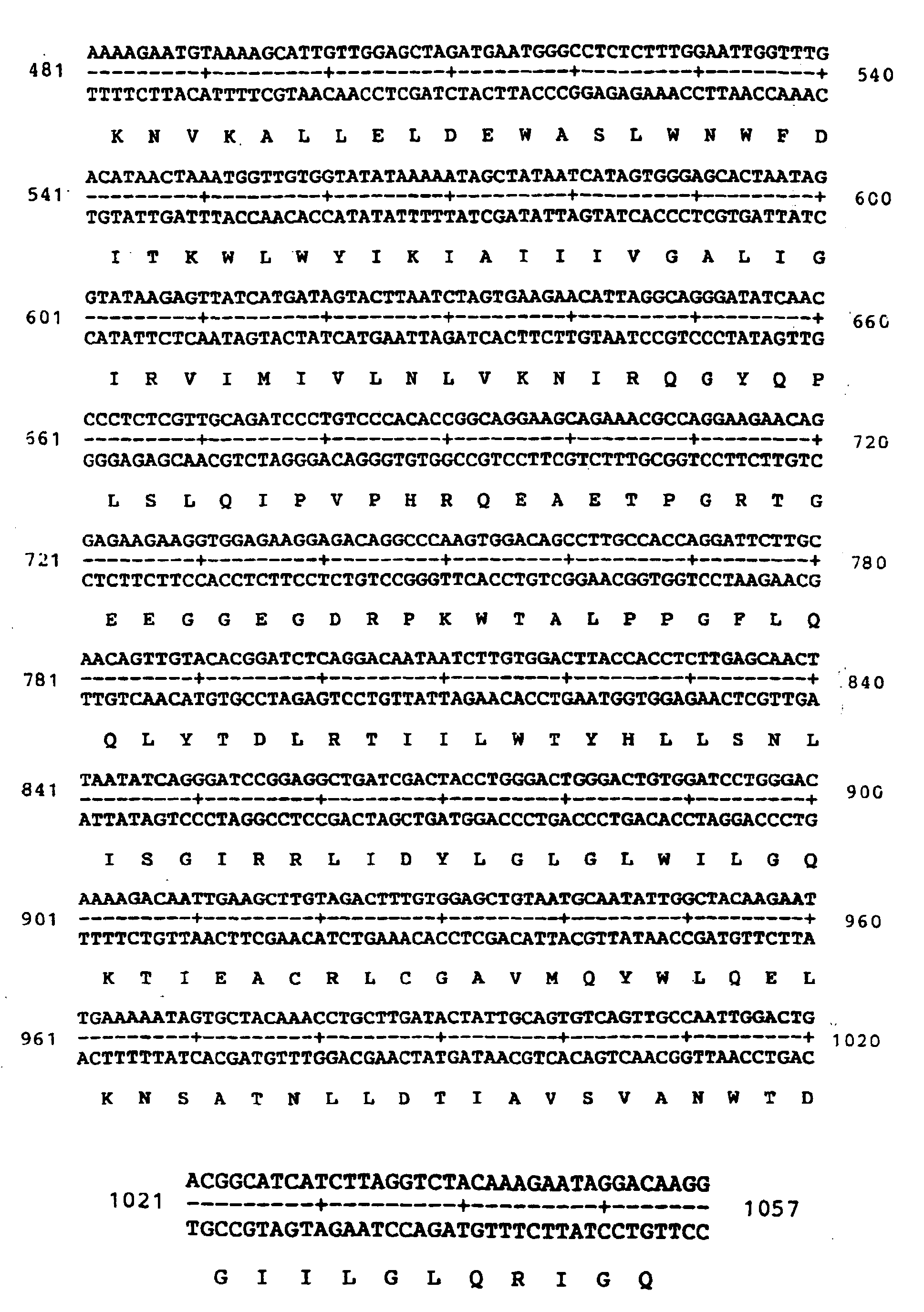

### Beispiel 8

Die gefundene Nucleotidsequenz aus Tabelle 3 wurde auf homologe Sequenzen in der GENEBANK-Datenbank (Release 72, Juni 1992) mit Hilfe des GCG-Computerprogramms (Genetic Computer Group, Inc. Wisconsin USA, Version 7.1, März 1992) untersucht. In dieser Datenbank sind die meisten der bis Juli 1992 bekannten Nucleotidsequenzen von Immundefizienzviren humanen Ursprungs und von Isolaten aus Primaten enthalten.

Die Nucleotidsequenz aus Tabelle 3 weist im besten Fall eine 62 %ige Homologie zu einem HIV 1-Isolat auf. Zu HIV 2 Isolaten ist die DNA aus Tabelle 5 zu 50 % homolog.

Die aus der Nucleotidsequenz aus Tabelle 3 abgeleitete Aminosäuresequenz wurde auf homologe Sequenzen in der SWISSPROT Protein-Datenbank (Release 22, Juni 1992) mit Hilfe des GCG-Computerprogramms untersucht. In dieser Datenbank sind die meisten der bis Juni 1992 bekannten Proteinsequenzen von Immundefizienzviren humanen Ursprungs und von Isolaten aus Primaten enthalten.

Die Aminosäuresequenz aus Tabelle 3 ist im besten Fall mit 54 % zu dem korrespondierenden Hüllproteinabschnitt eines Schimpansen-Isolates CIV (SIVcpz) homolog und zu 54,5 % zu dem HIV 1-Isolat Mal. Zu HIV 2-Hüllproteinen beträgt die Homologie mit der Aminosäuresequenz aus Tabelle 3 im besten Fall 34 % (Isolat HIV 2 D194).

Vergleicht man demgegenüber die gp 41-Aminosäuresequenz von HIV 1 mit den in der SWISSPROT-Datenbank vorhandenen HIV 1 gp 41-Sequenz, ergeben sich wie erwartet im besten Fall eine fast 100 %ige Homologie und im schlechtesten Fall eine 78 %ige Homologie.

Aufgrund dieser deutlichen strukturellen Unterschiede zwischen dem Sequenzbereich aus Tabelle 3 und dem korrespondierenden Abschnitt aus HIV 1 und HIV 2 scheint es sich bei dem Isolat MVP-5180/91 um eine HIV-Variante zu handeln, die sich von HIV 1 und HIV 2 deutlich strukturell abgrenzt. Möglicherweise ist MVP-5180/91 einer eigenen, von HIV 1 und HIV 2 sich abgrenzenden Gruppe von HIV-Viren zuzuordnen.

Das Peptid von Aminosäure 584-618 des HIV 1-Hüllproteinbereichs ist von besonderem serodiagnostischem Interesse (Numerierung nach Wain Hobson et al., Cell 40:9-17, 1985; Gnann et al., J. Inf. Dis. 156:261-267, 1987; Norrby et al., Nature, 329:248-250, 1987). Korrespondierende Aminosäurebereiche aus den Hüllproteinen von HIV 2 und SIV sind ebenfalls immundiagnostisch konserviert (Gnann et al., Science, S. 1346-1349, 1987). So werden Peptide aus diesem Hüllproteinbereich von HIV 1 und HIV 2 in vielen kommerziell erhältlichen HIV 1/2 Antikörper-Screeningtests als Festphasenantigene eingesetzt. Ungefähr 99 % der Anti-HIV 1 und Anti-HIV 2 positiven Seren können damit erfaßt werden.

Der korrespondierende Aminosäurebereich des MVP-5180/91-Hüllproteins (Tabelle 4) als auch das gesamte gp 41 dieses Isolates könnten serodiagnostisch insbesondere dann von Bedeutung sein, wenn Antiseren von HIV-infizierten Patienten in kommerziell erhältlichen Antikörper-Screening-Tests nur schwach oder überhaupt nicht reagieren würden. In diesen Fällen könnte eine Infektion mit einem MVP-5180/91 eng verwandten Virus vorliegen.

**Tab. 4:**

| | |
|---|---|
| 1 | RILAVERYLKDQQLLGIWGCSGKLICTTAVPWNAS |
| 2 | LQ L TLIQN R NL K Y S K T |
| | |
| 1 | HIV 1 Aminosäuresequenz aus gp41 |
| 2 | MvP5180-Sequenz aus gp41. Nur Unterschiede zu der HIV 1-Sequenz sind ausgedruckt. |

Das Peptid, das mit Hilfe der von MvP 5180 stammenden Information aufgefunden wurde, hat also die Aminosäuresequenz: RLQALETLIQNQQRLNLWGCKGKLICYTSVKWNTS.

Gegenstand der vorliegenden Erfindung ist daher auch die diagnostische Verwendung von Peptiden, die rekombinant oder synthetisch hergestellt werden können und die oben angegebene Sequenz oder Teilsequenz aufweisen, wobei die Teilsequenzen wenigstens 6 aufeinanderfolgende Aminosäuren, bevorzugt 9 und besonders bevorzugt 12 aufeinanderfolgende Aminosäuren aufweisen.

### Beispiel 9

### Klonierung des Gesamtgenoms des HIV-Isolates MvP5180

### a) Herstellung einer genomischen Bibliothek

Genomische DNA aus MvP5180-infizierten HUT78-Zellen wurde wie beschrieben isoliert.
300 µg dieser DNA wurde in einem Volumen von 770 µl mit 0,24 U des Restriktionsenzyms Sau3A für 45 min inkubiert.

Die dadurch nur partiell geschnittene DNA wurde anschließend über ein 0,7 %iges Agarosegel (low melting agarose, Nusieve) größenfraktioniert und Fragmente zwischen 10 und 21 kb ausgeschnitten. Die Agarose wurde für 10 min bei 70°C geschmolzen und mit demselben Volumen Puffer (1 x TBE, 0,2 M NaCl) versetzt. Anschließend folgte nach zweimaliger Phenol- und einmaliger Chloroformextraktion die Fällung der DNA durch Zugabe von 1/10 Vol 3 M Natriumacetatlösung (pH 5,9) und 2,5 Vol Ethanol bei -70°C für 10 min. Die gefällte DNA wurde abzentrifugiert, getrocknet und in einer Konzentration von 1 µg/µl in Wasser gelöst.
Die Ausbeute an größenfraktionierter DNA betrug etwa 60 µg. 5 µg dieser DNA wurden mit 1 U Alkalische Phosphatase im entsprechenden Puffer für 20 min bei 37°C inkubiert. Durch Abspaltung des 5'-terminalen Phosphatrestes wurden so multiple Insertionen von größenfraktionierter DNA vermindert. Die Phosphatasebehandlung wurde durch Phenolisierung gestoppt, die DNA wie oben gefällt und zusammen mit 1 µg des Vektors (2 DASH, BamHI geschnitten, Stratagene Nr.: 247611) in 6 µl Gesamtvolumen mit 2 Weiss-Units Lambda T4 Ligase für 12 Stunden bei 15°C ligiert. Nach erfolgter Ligation wurde die DNA mit Hilfe eines Verpackungskits (Gigapack II Gold, Stratagene Nr.: 247611) genau nach Angaben des Herstellers in Phagenhüllen verpackt.

### b) Radioaktive Markierung der DNA-Probe

Für die Markierung wurde der "Random Primed DNA Labeling Kit" von Boehringer Mannheim (Nr.: 713 023) eingesetzt. Markiert wurde das PCR-Produkt, welches wie in Beispiel 3 beschrieben mit den Primern sk68 und envb erhalten wurde. 1 µg dieser DNA wurde durch 2 x 5 min Kochen und anschließender Abkühlung in Eiswasser denaturiert. Zur Markierung wurden 50 mCi [α-³²P]-dCTP (NEN, Nr.: NEX-053H) zugegeben. Sonstige Zusätze wurden nach Herstellerangaben pipettiert. Nach einer Inkubation von 30 min bei 37°C erfolgte eine Fällung der nun radioaktiv markierten DNA.

### c) Screening der Phagen-Bibliothek

Zu 200 µl einer bei 30°C über Nacht angezogenen Kultur (Stamm SRB(P2) [Stratagene, Nr.: 247611] in LB-Medium, welches 10 mM MgSO₄, sowie 0,2 % Maltose enthielt) wurden 20000 pfu (Plaque forming units) der Bibliothek in 100 µl SM-Puffer (5,8 g NaCl, 2 g MgSO₄ 50 ml 1 M Tris. pH 7,5 und 5 ml einer 2 % Gelatinelösung in 1 l H₂O gelöst) gegeben, die Phagen 20 min bei 37°C an die Bakterien adsorbiert, mit 7,5 ml auf 55°C abgekühlter Top-Agarose gemischt und auf einer vorgewärmten Lb-Agarplatte von 14 cm Durchmesser verteilt. Nach etwa 8 Stunden erreichten die Plaques Konfluenz. Daraufhin wurden Nitrocellulosefilter für wenige Minuten auf die Platten gelegt und asymmetrische Markierungen angebracht. Nach vorsichtigem Abheben wurden die Filter für 2 min denaturiert (0,5 M NaOH, 1,5 M NaCl) und dann 5 min neutralisiert (0,5 M Tris, pH 8, 1,5 M NaCl). Nach anschließendem Backen der Filter bei 80°C für 60 min, konnten die Filter mit der Probe hybridisiert werden.
Zur Vorhybridisierung wurden die Filter in 15 ml Hybridisierungslösung (50 % Formamid, 0,5 % SDS, 5 x SSPE, 5 x Denhardt's Lösung und 0,1 mg/ml Lachssperma-DNA) pro Filter bei 42°C unter Schütteln 2-3 h inkubiert. Die [³²P]-markierten DNA-Proben wurden 2-5 min bei 100°C denaturiert, auf Eis abgekühlt, der Vorhybridisierungslösung zugesetzt und 12 Stunden bei 42°C hybridisiert. Anschließend wurden die Filter bei 60°C zunächst mit 2 x SSC/0,1 % SDS, dann mit 0,2 x SSC/0,1 % SDS gewaschen. Nach Trocknen der Filter wurden Hybridisierungssignale mit Hilfe des Röntgenfilms X-OMAT™AR (Kodak) detektiert.
Die Plaques, denen ein Signal zugeordnet werden konnte, wurden nach Elution in SM-Puffer in weiteren Verdünnungsschritten vereinzelt.
Der unten beschriebene Klon konnte nach dem Screening von 2 x 10⁶ Plaques identifiziert werden.

### d) Isolierung der Phagen-DNA und Subklonierung

Mit 10 µl eines Phageneluats in SM-Puffer, wurde eine Übernachtkultur des Wirtsstammes SRB (P2) so infiziert, daß nach zunächst dichtem Wachstum der Kultur nach etwa 6-8 h Lyse erfolgte. Von der lysierten Kultur wurden Zellreste durch zweimalige Zentrifugation bei 9000 g für 10 min abgetrennt. Anschließend wurden die Phagen durch Zentrifugation pelletiert (35000 g, l h), in 700 µl 10 mM MgSO₄ aufgenommen und solange phenolisiert, bis keine Proteininterphase mehr zu sehen war. Daraufhin wurde die Phagen-DNA gefällt, mit dem Restriktionsenzym EcoRI geschnitten und die daraus erhaltenen EcoRI-Fragmente in den Vektor Bluescript KS⁻ (Strategene, Nr.: 212208) subkloniert. Insgesamt wurden 4 Klone erhalten:

| **Plasmid** | **Beginn¹** | **Ende¹** |
|---|---|---|
| pSP1 | 1 | 1785 |
| pSP2 | 1786 | 5833 |
| pSP3 | 5834 | 7415 |
| pSP4 | 7660 | 9793 |

| | | |
|---|---|---|
| ¹bzgl. der folgenden Gesamtsequenz | | |

Das fehlende Stück zwischen Base 7416 und 7659 wurde durch PCR mit den Primern 157 (CCA TAA TAT TCA GCA GAA CTA G) und 226 (GCT GAT TCT GTA TAA GGG) erhalten. Als DNA-Template wurde die Phagen-DNA des Klons verwendet. Die Bedingungen für die PCR waren: 1.) Initiale Denaturierung: 94°C, 3 min, 2.) Amplifikation: 1,5 min 94°C, 1 min 56°C und 1 min 72°C für 30 Zyklen.
Die Sequenzierung der DNA erfolgte wie in Beispiel 4 beschrieben. Vom gesamten Genom wurde sowohl der Strang- als auch der Gegenstrang sequenziert. Bei allen EcoRI Schnittstellen wurde durch PCR mit Phagen-DNA des Klons als DNA-Template verifiziert, daß es sich an den Subklonübergängen jeweils um singuläre EcoRI-Schnittstellen handelt.

**Tab.5**

| **Die Lage der Gene der Virusproteine GAG, POL und ENV in der Gesamtsequenz von MvP5180** | | |
|---|---|---|
| **Gen** | **Start¹** | **Stop¹** |
| GAG | 817 | 2310 |
| POL | 2073 | 5153 |
| ENV | 6260 | 8887 |

| | | |
|---|---|---|
| 1.) Die Zahlen geben die Basenpositionen in der Gesamtsequenz von MvP5180/91 | | |

Die Gesamtsequenz von MvP5180/91 ist in Fig. 4 dargestellt.

### Beispiel 10

### Abgrenzung der Gesamtsequenz von MvP5180/91 von anderen HIV1-Isolaten

Grundlage für die folgenden Sequenzvergleiche war die Datenbanken Genbank Release 75 von 2.93, EMBL 33 von 12.92 und Swissprot 24 von 1.93. Homologievergleiche erfolgten mit der GCG-Software (Version 7.2, 10.92. der Genetics-Computer-Group, Wisconsin).

Zunächst wurden auf Aminosäureebene die Sequenzen von GAG, POL und ENV mit dem Programm "Wordsearch" mit der Datenbank verglichen. Die 50 besten Homologen wurden mit dem Programm "Pileup" jeweils untereinander verglichen. Daraus geht deutlich hervor, daß MvP5180/91 in den HIV1-Stammbaum fällt, dort aber sehr früh, sogar noch vor dem Schimpansenvirus SIVcpz abzweigt, also eine neue Subfamilie von HIV1 repräsentiert. Um Zahlenwerte für die Homologien zu erhalten; wurde mit dem Programm "Gap" MvP5180 mit den jeweils am besten passenden HIV1, HIV2 und SIV-Sequenzen und zusätzlich mit der SIVcpz-Sequenz verglichen.

**Tab.6**

| **Homologiewerte der Aminosäuresequenz von GAG, POL und ENV des MvP5180/91-Isolates** | | | | | | | | |
|---|---|---|---|---|---|---|---|---|
| **GAG** | SIVcpz | 70,2% | HIV1u² | 69,9% | HIV2d³ | 53,6% | SIV1a⁴ | 55,1% |
| | | 83,6% | | 81,2% | | 71,3% | | 71,3% |
| **POL** | SIVcpz | 78,0% | HIV1u² | 76,1% | HIV2d³ | 57,2% | SIVgb⁵ | 57,7% |
| | | 88,0% | | 86,8% | | 71,9% | | 74,6% |
| **ENV** | SIVcpz | 53,4% | HIV1h¹ | 50,9% | HIV2d³ | 34,4% | SIVat⁶ | 34,4% |
| | | 67,1% | | 67,2% | | 58,7% | | 57,8% |

| | | | | | | | | |
|---|---|---|---|---|---|---|---|---|
| ¹h=hz321/Zaire, | | | | | | | | |
| ²u=u455/Uganda, | | | | | | | | |
| ³d=jrcst, | | | | | | | | |
| ⁴a=agm155, | | | | | | | | |
| ⁵gb=gb1 | | | | | | | | |
| ⁶at=agm | | | | | | | | |

Der obere Zahlenwert drückt die Identität, der untere die Ähnlichkeit beider Sequenzen aus.
Weiter wurde die Datenbank mit "Wordsearch" und "Gap" auf Nukleotidebene durchsucht. Die Homologiewerte für die jeweils besten "matches" sind in Tabelle 7 zusammengefaßt.

**Tab.7**

| **Homologiewerte der Nukleotidsequenz von MvP5180/91** | | | | |
|---|---|---|---|---|
| | **HIV1** | | **HIV2** | |
| **gag** | HIVelicg | 70,24 % | HIV2bihz | 60,0 % |
| **pol** | HIVmal | 75,0 % | HIV2cam2 | 62,9 % |
| **env** | HIVsimi84 | 59,7 % | HIV2gha | 49,8 % |

### Beispiel 11

### Beschreibung der PCR Amplifizierung, Klonierung und Sequenzierung des gag Gens des HIV 5180 Isolates

Um die im Laufe der Virusvermehrung auftretenden Spontanmutationen darzustellen, wurde ein Teil des Virusgenoms mit der PCR-Technik kloniert und die so erhaltene DNS-Sequenz verglichen mit der Sequenz gemäß Fig. 4.

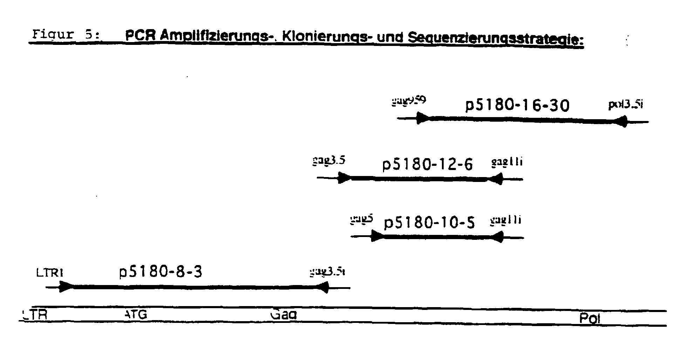

Die gag Sequenz wurde vom LTR ("long terminal repeat", LTR1 primer) des linken Endes des MvP 5180 Genomes bis in das pol (Polymerase Gen, pol3.5i primer) hinein überlappend kloniert. Die Klonierungsstrategie ist in Fig. 5 schematisch dargestellt.
Die PCR Reaktionen wurden mit den u.g. DNA Primern, deren Sequenzen von der HIV-1 Konsensus-Sequenz abgeleitet wurden, durchgeführt. Die Sequenzierungen erfolgten mit Hilfe der Dideoxykettenabbruchmethode.
Die für das MvP 5180 gag Gen kodierende Sequenz erstreckt sich von Nukleotid 817 (A des ATG Startkodons) bis Nukleotid 2300 (A des letzten Kodons) (Sequ. ID No. 47-53).

| | |
|---|---|
| LTR1: | 5' - CTA GCA GTG GCG CCC GAA CAG G -3' |
| gag3.5: | 5' - AAT GAG GAA GCU GCA GAU TGG GA -3' (U=A/T) |
| gag 3.5i: | 5' - TCC CAU TCT GCU GCT TCC TCA TT -3' (U=A/T) |
| gag5: | 5' - CCA AGG GGA AGT GAC ATA GCA GGA AC -3' |
| gag959: | 5' - CGT TGT TCA GAA TTC AAA CCC -3' |
| gag11i: | 5' - TCC CTA AAA AAT TAG CCT GTC -3' |
| po13.5i: | 5' - AAA CCT CCA ATT CCC CCT A -3' |

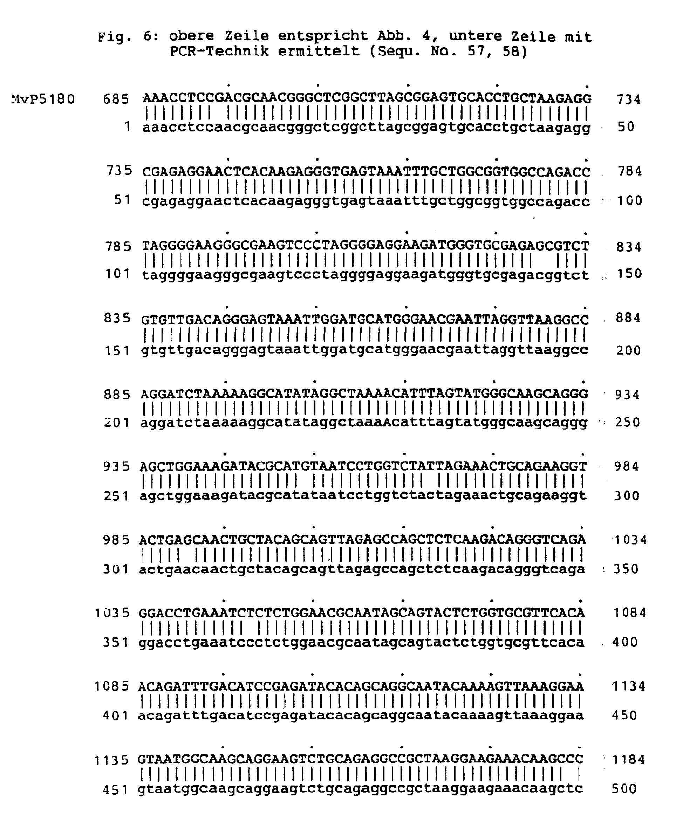

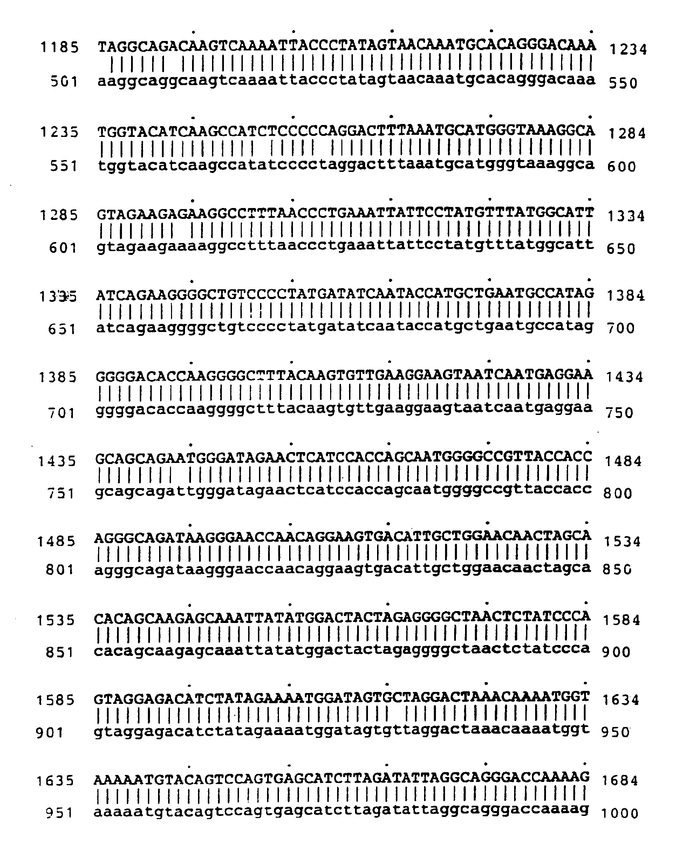

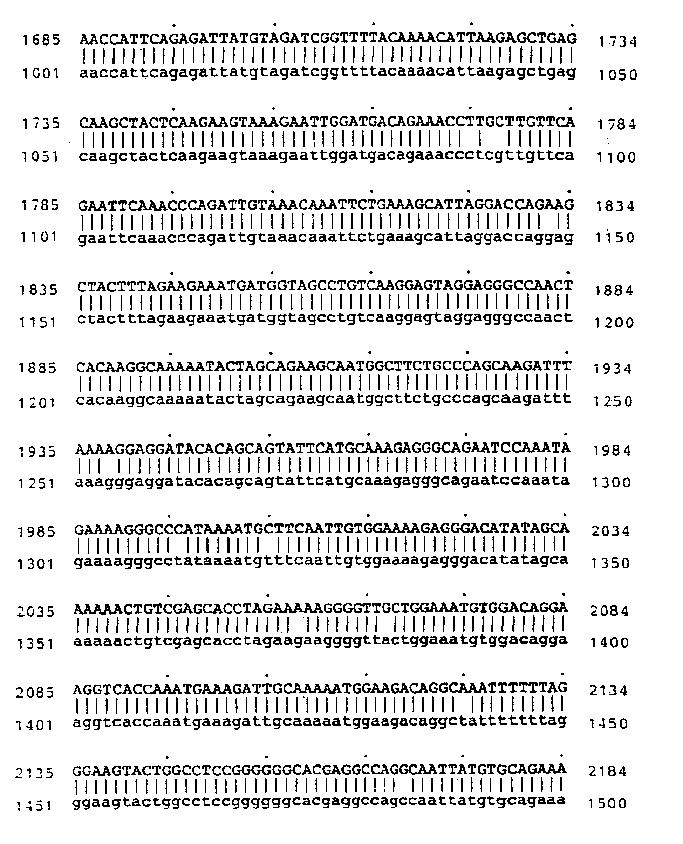

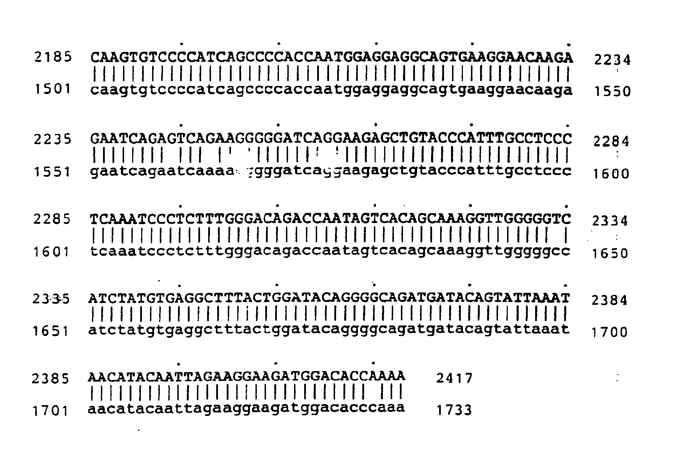

Die bei der PCR-Technik erhaltene DNS-Sequenz wurde der in Figur 4 dargestellten DNS-Sequenz gegenübergestellt. Ein Vergleich der beiden DNS-Sequenzen ist in Figur 6 dargestellt. Hierbei wurde festgestellt, daß sich die Nukleotide in ca. 2 % voneinander unterscheiden, obwohl es sich um dasselbe Virus handelt. In Fig. 6 stellt jeweils die obere Zeile die DNS-Sequenz dar, die in Fig. 4 dargestellt ist und die untere Zeile stellt die mit PCR-Technik erhaltene DNS-Sequenz dar.

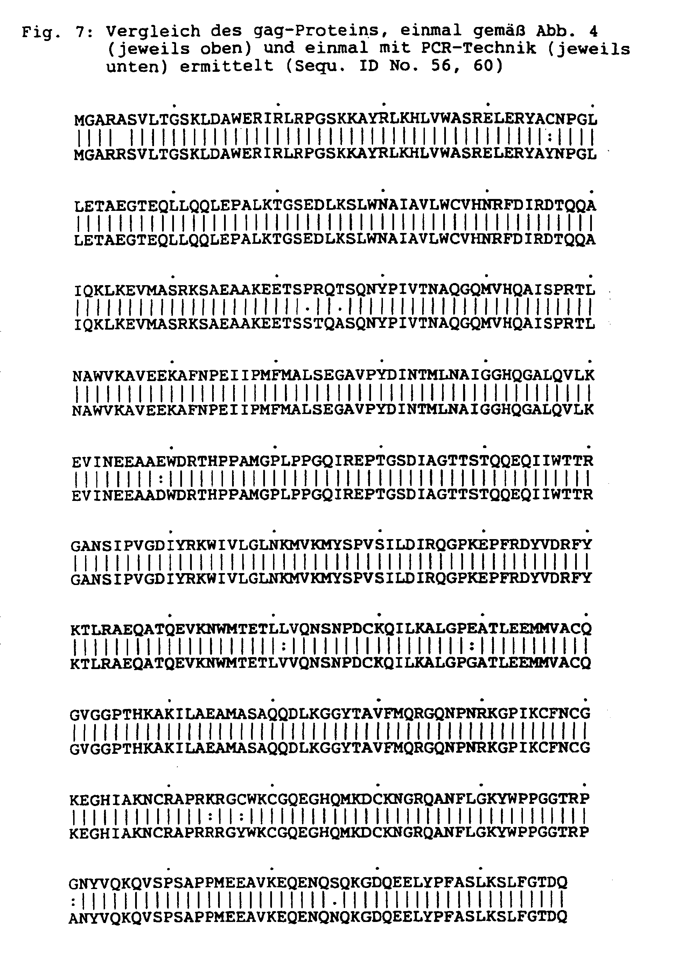

Weiterhin wurde die Aminosäuresequenz des mit PCR-Technik ermittelten Proteins gag der Aminosäuresequenz des aus Fig. 4 abgeleiteten entsprechenden Proteins gegenübergestellt. Dabei wurde ein Unterschied der Aminosäure von ca. 2,2 % ermittelt. Der Vergleich ist in Fig. 7 dargestellt, wobei die untere Zeile jeweils die Aminosäuresequenz darstellt, die von der mit PCR-Technik erhaltenen Sequenz abgeleitet wurde.

### Beispiel 12

Es wurde die Sequenz des erfindungsgemäßen Virus MvP 5180 verglichen mit den Konsensus-Sequenzen von HIV1 und HIV2 und soweit bekannt mit der Sequenz von ANT-70 (WO 89/12094).

Dabei wurden folgende Ergebnisse erhalten:

**Tab.8**

| Genort | abweichende Nukleotide | Zahl der Nukleotide | % Homologie (genähert) |
|---|---|---|---|
| LTR | 207 | 630 | HIV-1 67 % |
| | 308 | | HIV-2 51 % |
| | 115 | | ANT 70 82 % |
| GAG | 448 | 1501 | HIV-1 70 % |
| | 570 | | HIV-2 62 % |
| POL | 763 | 3010 | HIV-1 74 % |
| | 1011 | | HIV-2 66 % |
| VIF | 183 | 578 | HIV-1 68 % |
| | 338 | | HIV-2 42 % |
| ENV | 1196 | 2534 | HIV-1 53 % |
| | 1289 | | HIV-2 49 % |
| NEF | 285 | 621 | HIV-1 54 % |
| | 342 | | HIV-2 45 % |
| total | 3082 | 8874 | HIV-1 65 % |
| | 3858 | | HIV-2 56 % |

In der obigen Tabelle bedeuten "HIV-1" Konsensus-Sequenzen von HIV-1 Viren; "HIV-2" Konsensus-Sequenzen von HIV-2 Viren; ANT-70 aus der WO 89/12094 bekannte Teilsequenz eines als HIV-3 bezeichneten Virus.

Offenbart werden daher Viren, DNS-Sequenzen, und die diagnostische Verwendung von Aminosäuresequenzen sowie Teilsequenzen davon, die eine solche Homologie mit der in Fig. 4 dargestellten Sequenz aufweisen, bezogen auf die Genorte, daß höchstens die in Tabelle 9 angegebenen Anteile, ausgedrückt in %-Werten, unterschiedlich sind.

**Tab.9**

| **Homologie bezogen auf Genorte, ausgedrückt als maximale Unterschiede** | | | |
|---|---|---|---|
| **Genort** | **Unterschiede** | **bevorzugte Unterschiede** | **besonders bevorzugte Unterschiede** |
| LTR | 17 % | 15 % | 10 % |
| GAG | 29 % | 28 % | 14 % |
| POL | 25 % | 24 % | 12 % |
| VIF | 31 % | 30 % | 15 % |
| ENV | 46 % | 45 % | 22 % |
| NEF | 16 % | 12 % | 10 % |

Die in Tabelle 9 angegebenen Homologiewerte in % bedeuten, daß bei einer Gegenüberstellung der Sequenz gemäß Fig. 4 mit einer Sequenz eines anderen Virus höchstens ein den oben angegebenen Prozentwerten entsprechender Anteil der Sequenz unterschiedlich sein darf.

### Beispiel 13

### V3-loop / V3-Schlaufe

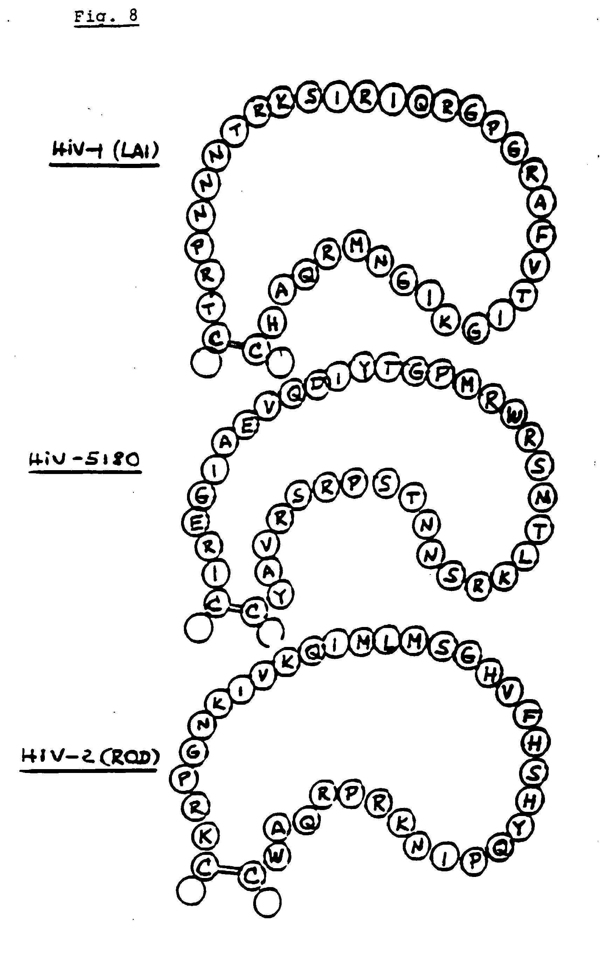

Diese Schlaufe ist die hauptneutralisierende Region im HIV und die Dokumentation der immunologischen Spezifitäten der Region sind in der Figur 8 zusammengefaßt. Dies ist eine Kopie aus AIDS aus einer Arbeit von Peter Nara (1990).
Die V3-Schlaufe ist dann in Aminosäuren-Ebene aufgezeichnet und mit dem IIIB Virus jetzt LAI und dem ersten HIV-2 Isolat (ROD) verglichen. Einzelne Aminosäuren an der Cystin-Brücke sind konserviert. Während die Krone von HIV-1 GPGR oder GPGQ ist und die von HIV-2 GHVF ist die Krone des MvP5180/91 gebildet aus den Aminosäuren GPMR. Das Motiv mit dem Methionin ist bisher nicht beschrieben worden und unterstreicht die Individualität des MvP 5180/91.

Nachdem die Nukleinsäuresequenz des Virus ermittelt war, wurde der V3-Loop-Bereich mit Hilfe der PCR-Technik unter Verwendung geeigneter Primer amplifiziert. Hierbei konnten Mutationen beobachtet werden, insbesondere eine Veränderung des Metheonin-Kodons (ATG) zu einem Leucin-Kodon (CTG).

Nachfolgend wird eine Gegenüberstellung der von der klonierten Nukleinsäure abgeleiteten Aminosäuresequenz und der Sequenz gegeben, die nach Amplifizierung mittels PCR-Technologie erhalten wurde (Sequ. ID No. 54-55):
MvP 5180 (kloniert):
   CIREGIAEVQDIYTGPMRWRSMTLKRSNNTSPRSRVAYC
MvP 5180 (PCR-Technik):
   CIREGIAEVQDLHTGPLRWRSMTLKKSSNSHTQPRSKVAYC

### Beispiel 14

Um zu zeigen, daß mit Hilfe des erfindungsgemäßen Virus MvP 5180 bzw. davon abgeleiteten Antigenen auch solche Seren als HIV-1 positiv nachgewiesen werden können, die bei Verwendung eines normalen HIV-1+2 Screening-Tests nicht erfaßt werden können, wurden verschiedene Seren von Patienten aus Kamerun im EIA-Test überprüft.

Bei einer Studie in Kamerun wurden 156 Anti-HIV-1-positive Seren überprüft. Bei zwei dieser Seren wurden erhebliche, diagnostisch relevante Unterschiede beobachtet. Bei der nachfolgenden Tabelle 10 werden die gemessenen Extinktionen angegeben. CAM-A bzw. CAM-B stehen für die Seren verschiedener Patienten.

**Tabelle 10**

| Patientenseren | MvP 5180-EIA | HIV-1 + HIV-2 EIA |
|---|---|---|
| CAM-A | 2.886 | 1.623 |
| CAM-B | 1.102 | 0.386 |

Der Cutoff für beide Tests betrug 0.300.

In einer weiteren Studie mit 47 Anti-HIV-1-positiven Seren aus Kamerun fielen zwei Seren besonders auf. Eines davon (93-1000) stammt von einem wenig symptomatischen, das andere (93-1001) von einem AIDS-kranken Patienten. In der folgenden Tabelle 11 werden die Extinktionswerte der beiden EIA-Tests einander gegenübergestellt:

**Tabelle 11**

| Patientenserum | MvP 5180-EIA | HIV-1 + HIV-2 EIA |
|---|---|---|
| 93-1000 | > 2.5 | 1.495 |
| 93-1001 | 0.692 | 0.314 |

Auch hier betrug der Cutoff 0.3. Die Extinktionswerte des Patienten 93-1001 zeigen, daß der normale HIV-1 + HIV-2 EIA versagen kann, wohingegen durch Einsatz des erfindungsgemäßen Antigens ein klarer Nachweis möglich ist.

## Patentansprüche

1. Immunschwäche-Virus der HIV-Gruppe, das bei der European Collection of Animal Cell Cultures (ECACC) unter der Nr. V 920 92 318 mit der Bezeichnung MVP-518/91 hinterlegt wurde, sowie Varianten des Virus, wobei eine Variante eine Sequenz aufweist, die der Tabelle 3 entspricht oder zu dieser Sequenz homolog ist, wobei die Homologie mehr als 66 % bezogen auf die Nukleotidsequenz beträgt.

2. Virus gemäß Anspruch 1, wobei die Homologie mehr als 75 % beträgt.

3. Virus gemäß Anspruch 1, wobei die Homologie mehr als 85 % beträgt.

4. Testkit zum Nachweis von Antikörpern gegen Immunschwäche verursachende Viren, die einer unabhängigen Gruppe von HIV-Viren gemäß Anspruch 1 bis 3 angehören, worin ein Antigen eingesetzt wird, das eine Aminosäuresequenz oder Aminosäureteilsequenz aufweist, die von einem derartigen Virus abgeleitet ist, und, die der Tabelle 3 oder einer Teilsequenz davon entspricht und die Teilsequenz wenigstens 6 Aminosäuren aufweist.

5. Testkit nach Anspruch 4, wobei das Antigen die Aminosäuresequenz RLQALETUONQQRLNLWGCKGKLICYTSVKWNTS oder eine Teilsequenz davon mit wenigstens 6 aufeinanderfolgenden Aminosäuren aufweist.

6. Testkit nach einem der Ansprüche 4 bis 5, wobei das Antigen rekombinant hergestellt wurde.

7. Testkit nach einem der Ansprüche 4 bis 5, wobei das Antigen synthetisch hergestellt wurde.

8. Testkit gemäß einem der Ansprüche 4 bis 7, das ein Western Blot ist.

9. Testkit gemäß einem der Ansprüche 4 bis 7, das ein ELISA-Test oder ein Fluoreszenzantikörper-Nachweistest ist.

10. Verwendung eines von dem bei der European Collection of Animal Cell Cultures (ECACC) unter der Nr. V 920 92 318 mit der Bezeichnung MVP-5180191 hinterlegten Virus abgeleiteten Antigens zum Nachweis von Retroviren, die Immunschwäche verursachen und die einer unabhängigen Gruppe von HIV-Viren gemäß Anspruch 1 angehören.

11. Verwendung eines Antigens nach Anspruch 10, wobei das Antigen eine Aminosäuresequenz aufweist, die der Tabelle 3 oder einer Teilsequenz davon entspricht, und die Teilsequenz wenigstens 6 Aminosäuren aufweist.

12. Verwendung eines Antigens nach einem der Ansprüche 10 oder 11, wobei das Antigen die Aminosauresoquenz RLQALETLIQNQQRLNLWGCKGKUCYTSVKWNTS oder eine Teilsequenz davon mit wenigstens 6 aufeinanderfolgenden Aminosäuren aufweist.

## Claims

1. An immunodeficiency virus of the HIV group, which has been deposited with the European Collection of Animal Cell Cultures (ECACC) under No. V 920 92 318 with the designation MVP-5180/91, as well as variants of this virus, wherein one variant has a sequence corresponding to table 3 or being homologous to this sequence, wherein the homology is higher than 66% based on the nucleotide sequence.

2. The virus as claimed in claim 1, wherein the homology is higher than 75%.

3. The virus as claimed in claim 1, wherein the homology is higher than 85%.

4. A test kit for detecting antibodies against viruses which cause immunodeficiency, belonging to an independent group of HIV viruses as claimed in claims 1-3, wherein an antigen is used having an amino acid sequence or a constituent amino acid sequence which is deduced from such a virus, and which corresponds to table 3 or a constituent sequence thereof and the constituent sequence has at least 6 amino acids.

5. The test kit as claimed in claim 4, wherein the antigen has the amino acid sequence RLQALETLIQNQQRLNLWGCKGKLICYTSVKWNTS or a constituent sequence thereof having at least 6 consecutive amino acids.

6. The test kit as claimed in any one of claims 4 to 5, wherein the antigen was prepared recombinantly.

7. The test kit as claimed in any one of claims 4 to 5, wherein the antigen was prepared synthetically.

8. The test kit as claimed in any one of claims 4 to 7, which is a Western blot.

9. The test kit as claimed in any one of claims 4 to 7, which is an ELISA test or a fluorescence-antibody detection test.

10. Use of an antigen deduced from a virus, which has been deposited with the European Collection of Animal Cell Cultures (ECACC) under No. V 920 92 318 with the designation MVP-5180/91 for detecting retroviruses which cause immunodeficiency and which belong to an independent group of HIV viruses as claimed in claim 1.

11. Use of an antigen as claimed in claim 10, wherein the antigen has an amino acid sequence corresponding to table 3 or a constituent sequence thereof and the constituent sequence has at least 6 amino acids.

12. Use of an antigen as claimed in claim 10 or 11, wherein the antigen has the amino acid sequence RLQALETLIQNQQRLNLWGCKGKLICYTSVKWNTS or a constituent sequence thereof having at least 6 consecutive amino acids.

## Revendications

1. Virus de l'immunodéficience du groupe VIH qui fut déposé auprès de l'European Collection of Animal Cell Cultures (ECACC) sous le numéro V 920 92 318 avec la désignation MVP-5180/91, ainsi que des variantes du virus, où une variante présente une séquence qui correspond à celle présentée dans le tableau 3, ou qui est homologue de cette séquence, où l'homologie est superieure à 66%, par rapport à la séquence des nucléotides.

2. Virus suivant la revendication 1, où l'homologie est supérieure à 75%.

3. Virus suivant la revendication 1, où l'homologie est supérieure à 85%.

4. Trousse d'essai pour la détermination d'anticorps contre les virus qui causent l'immunodéficience, qui est appartiennent à un groupe indépendant des virus-VIH suivant les revendications 1 à 3, où l'on met en oeuvre un antigène qui présente une séquence d'aminoacides ou une séquence partielle d'aminoacides, qui dérive d'un virus de ce genre, et qui correspond à celle présentée dans le tableau 3 ou à une sequence partielle de celle-ci et la sequence partielle présente au moins 6 aminoacides.

5. Trousse suivant la revendication 4, où l'antigène présente la séquence d'aminoacides RLQALETLIQNQQRLNLWGCKGKLICYTSVKWNTS ou une séquence partielle de celle-ci avec au moins 6 aminoacides successifs.

6. Trousse suivant l'une quelconque des revendications 4 à 5, où l'antigène recombiné a été préparé.

7. Trousse suivant l'une quelconque des revendications 4 à 5, où l'antigène a été préparé par voie synthétique.

8. Trousse suivant l'une quelconque des revendications 4 à 7, qui est un Western Blot (immunotransfert).

9. Trousse suivant l'une quelconque des revendications 4 à 7, qui est un test ELISA ou un test de détermination d'anticorps par fluorescence.

10. Utilisation d'un antigène dérivé d'un virus qui fut déposé auprès de l'European Collection of Animal Cell Cultures (ECACC) sous le numéro V 920 92 318 avec la designation MVP-5180/91, pour la détermination de retrovirus du genre de ceux qui provoquent l'immunodéficience et qui appartiennent à un groupe indépendant de virus-VIH suivant la revendication 1.

11. Utilisation d'un antigène suivant la revendication 10, où l'antigène présente une séquence d'aminoacides qui correspond à celle présentée dans le tableau 3 ou à une sequence partielle de celle-ci et la séquence partielle présente au moins 6 aminoacides.

12. Utilisation d'un antigène suivant l'une quelconque des revendications 10 ou 11, où l'antigène présente la séquence d'aminoacides RLQALETLIQNQQRLNLWGCKGKLICYTSVKWNTS ou une séquence partielle de celle-ci avec au moins 6 aminoacides successifs.
